# EUROPÄISCHE PATENTSCHRIFT

(11) **EP 1 585 445 B1**
(45) Veröffentlichungstag und Bekanntmachung des Hinweises auf die Patenterteilung: **28.09.2011**
(21) Anmeldenummer: 04703781.7
(22) Anmeldetag: 21.01.2004
(51) Int. Cl.: A61B 17/00

(54) **IMPLANTIERBARE EINRICHTUNG**
IMPLANTABLE DEVICE
DISPOSITIF IMPLANTABLE

(30) Priorität: 21.01.2003 DE 10302447
(43) Veröffentlichungstag der Anmeldung: 19.10.2005
(73) Patentinhaber: pfm medical ag, 50966 Köln (DE)
(72) Erfinder: FREUDENTHAL, Franz, La Paz (BO)
(74) Vertreter: Polypatent
(86) Internationale Anmeldenummer: PCT/EP2004/000451
(87) Internationale Veröffentlichungsnummer: WO 2004/064671

(56) Entgegenhaltungen:
- EP-A- 0 857 471
- EP-A- 1 210 919
- WO-A-00/44308
- WO-A-98/02100
- WO-A-98/47430
- WO-A-2004/047681
- DE-A- 10 118 944
- US-A- 5 876 445
- US-A1- 2002 193 827

## Beschreibung

Die Erfindung betrifft eine implantierbare Einrichtung zur Verwendung im menschlichen und/oder tierischen Körper zum Verschluss oder Teilverschluss von Defektöffnungen, Hohlräumen. Organwegen etc. oder zum Schaffen einer definierten Verbindungsöffnung zwischen Wandungen. Organen, Hohlräumen etc., mit einer Tragstruktur, die in einem ersten Betriebszustand (Primärform) ein großes Verhältnis von Länge zu Querausdehnung entlang einer Achse und in einem zweiten Betriebszustand (Sekundärform) ein kleineres Verhältnis von Länge zu Querausdehnung entlang der Achse aufweist, wobei die Tragstruktur einen proximalen und einen distalen Abschnitt aufweist.

Implantate sind im Stand der Technik bekannt. Beispielsweise ist in der US 5.846.261 eine zusammenfaltbare medizinische Einrichtung offenbart, die ein textiles Flächengebilde aus Metall aufweist mit einem Rücksprung an proximalem und distalem Ende eines vorgefertigten Aufbaus, wobei Befestigungseinrichtungen an proximalem und distalem Ende an dem textilen Flächengebilde aus Metall auf dem Rücksprung vorgesehen sind. Im ausgestreckten Zustand der medizinischen Einrichtung weist diese eine Glockenform auf. Das textile Flächengebilde ist aus einer Vielzahl von Drähten gebildet, die jeweils miteinander verwoben werden und endseitig zusammengeführt sind. Auch gemäß der US 5.725.552 ist ein verwobenes textiles Metallflächengebilde gebildet, wobei am proximalen und distalen Ende jeweils eine Befestigungseinrichtung aufgebracht ist. Die einzelnen Metall stränge bzw. -litzen der Vielzahl von Metall strängen bzw. -litzen wird in dieser Befestigungseinrichtung zusammengeführt. Im ausgestreckten Zustand bildet sich wiederum eine Glockenform aus. Gemäß der US 6,368.339 B1 sowie der US 6.123.715 wird ein Verfahren zum Herstellen einer solchen medizinischen Einrichtung beschrieben, die in einem Kanal bzw. Hohlraum in dem Körper eines Patienten entfaltet werden kann. In einer zusammengefalteten Ausbildung kann die Einrichtung durch einen Katheter hindurchgeführt werden, um an die Stelle im Körper des Patienten zu gelangen, an der sie entfaltet werden soll. Bei diesem Verfahren wird zunächst ein textiles Flächengebilde aus einer Vielzahl von Strängen oder Litzen gebildet, die zueinander entsprechend ausgerichtet sind und aus einem wärmebehandelbaren Material bestehen, um eine gewünschte Form einzuprägen. Anschließend wird das textile Flächengebilde im Wesentlichen an die innere Oberfläche eines Formgebungselementes angepasst, wodurch der ausgestreckte Zustand der Einrichtung definiert wird. Dann erfolgt eine Wärmebehandlung des textilen Flächengebildes in dem ausgestreckten Zustand. Anschließend wird das textile Flächengebilde von dem Formgebungselement entfernt. Die Stränge bzw. Litzen des textilen Flächengebildes sind wiederum auf einen gemeinsamen Endpunkt zusammengeführt am jeweiligen distalen und proximalen Ende der medizinischen Einrichtung. Ein entsprechendes Verfahren ist auch in der korrespondierenden EP 1 210 919 A2 offenbart.

Die vorstehend genannten Implantate dienen unter anderem zur Behandlung von Gefäßerkrankungen, wobei Gefäßdefekte durch minimalinvasive Chirurgie behandelt werden. Es wird dabei nicht die zu behandelnde Stelle direkt durch eine Operation geöffnet, sondern Instrumente und Implantate durch verhältnismäßig kleine Einschnitte im Leistenbereich oder im Bauchraum eingebracht. Die Implantate werden insbesondere in der Kardiologie zur Behandlung über Katheter in das Gefäßsystem eingeführt, insbesondere über die großen Beinadern. Bei der Behandlung von Septumdefekten des Herzens bietet die interventionelle Behandlung unter anderem den Vorteil, dass nicht mehr der Brustkorb geöffnet und das schwer stillzulegende und empfindliche Herz aufgeschnitten zu werden braucht.

Weitere Implantate und Kathetersysteme zur Platzierung eines solchen Implantats sind beispielsweise in der WO 97/28744 beschrieben, ebenso in der US 5.108.420 A. DE 42 22 291 A1, DE 28 22 603 A, WO 96/01591. WO 93/13712. WO 95/27448. US 5.433.727 A, EP 0 474 887 A1. In der WO 93/13712 wird ein Implantat zum Verschluss von Septumdefekten beschrieben, das im implantierten Zustand eine Doppelkonus- oder Doppelscheibenkonfiguration annimmt, wobei jeweils die äußeren Strukturen aus nicht direkt miteinander verbundenen Drahtelementen gebildet sind. Diese sind mit Stoffsegeln bespannt, wobei die Stoffsegel in einem dem zu verschließenden Defekt entsprechenden Radius miteinander vernäht sind. Nachteilig ist an diesem System, dass das aus einer Mehrzahl von Bauelementen aufgebaute Implantat einen hohen Montageaufwand erfordert.

In der WO 95/27448 ist ein Implantat beschrieben, das als Venenfilter eingesetzt und als tragende Struktur für einen Septumverschluss verwendet werden soll. Dabei wird aus einer Reihe von Einzelgeräten ein relativ langgestreckter Doppelkonus gebildet, wobei in einer Ausführungsform die Konen zueinander gerichtet sind nach Art eines Knochens und in einer weiteren Ausführungsform die Konen gleichgerichtet sind, ähnlich einem Fliegenpilz.

Aus der US 5.433.727 A ist ein Implantat bekannt, bei dem eine Art Schirm vor einen Septumdefekt gesetzt wird und durch den Defekt hindurch mit einem Gegenverschluss gesichert wird, der im Wesentlichen aus vier aus jeweils einem Draht hergestellten Schlaufen gebildet wird, die sich beim Ausstoßen aus einem Katheter entfalten und ein Durchrutschen des Implantats zu der Schirmseite verhindern sollen.

Die EP 0 474 887 A1 offenbart ein Implantat, bei dem zwei runde oder in sonstiger Weise polygonal geformte Dichtsegel, die durch jeweils ein umlaufendes nachgiebiges Rahmenelement aufgespannt werden, unter anderem über eine Vielzahl von Fäden verbunden sind, die zum Platzieren des Implantats durch den Katheter hindurch angezogen werden müssen. Zur Lagesicherung der beiden Segel wird ein zentraler Schnappverschluss vorgesehen. Aufgrund des sehr hohen Manipulationsaufwandes ist das in dieser Druckschrift beschriebene Implantat sehr schwierig zu platzieren und erfordert zudem eine komplizierte und daher sehr fehleranfällige Montage.

Gemäß der WO 97/28744 wird ein Implantat beschrieben, das sich beim Ausstoßen aus dem Katheter selbständig aufgrund einer eingeprägten Sekundärstruktur entfaltet und durch elastische Kräfte selbst den Abmessungen des Defekts in einem weiten Rahmen anpasst. Die eingeprägte Struktur klemmt sich nach Art einer Doppelscheibe nach beiden Seiten des Defekts gegen den umgebenden Bereich. Das Implantat ist aus einer Reihe drahtförmiger Elemente gebildet, die durch geeignete Fügeverfahren, wie Ultraschallschweißen oder Hartlöten miteinander verbunden sind. Außerdem ist das Implantat mit einer Bespannung versehen, die an den drahtförmigen Elementen befestigt ist.

Aus der DE 100 00 137 A1 ist ein Implantat bekannt, das in der Sekundärform in etwa die Form einer Doppelscheibe annimmt mit einem proximalen Scheibenelement und einem distalen Scheibenelement, wobei die Umgebung einer Defektöffnung zwischen den Scheibenelementen aufgenommen wird, wobei die Tragstruktur aus einem über einen Teil seiner Länge geschlitzten Rohr gebildet ist. Entlang dem geschlitzten Teil der Länge des Rohres sind Streifen gebildet, deren Breite variiert werden kann. Proximales und distales Ende der Tragstruktur sind weiterhin als Röhrchen ausgebildet, also dort die einzelnen Streifen zusammengeführt.

Gemäß der DE 196 04 817 A1 wird vorgeschlagen, ein aus einer Mehrzahl von drahtförmigen Elementen aufgebautes Implantat vorzusehen, das an einem Ende an einer Verbindungsstelle der einzelnen Drähte zur exakteren Positionierung im Körper des Patienten eine Einrichtung zum Deplatzieren aufweist. Diese Einrichtung zum Deplatzieren kann beispielsweise die Form einer Kugel aufweisen, ggf. mit Schlaufe zur Aufnahme eines Führungsdrahtes, damit der Schirm besser rechtwinklig zur Septumdefektwand positioniert und implantiert werden kann, auch wenn der Katheter nicht rechtwinklig zur Wand angeordnet ist. Am anderen Ende der Drähte bzw. des Drahtgerüstes sind die drahtförmigen Elemente beispielsweise durch eine weitere Kugel, eine Drahtverdrehung mit oder ohne Ösen, durch Löten, Schweißen, Kleben, Nähen, durch einen Faden, eine Buchse oder Ösen mit oder ohne Ring miteinander verbunden.

Die WO 98/02100 offenbart einen helikal gewickelten Stent zum Verschließen des Ductus Arteriosus mit einem Draht, der zum Einführen in den menschlichen Körper in einem ausgestreckten Zustand (Primärform) durch einen Katheter eingeführt werden kann und innerhalb des Körpers einen verschließenden Zustand annimmt (Sekundärform), in dem der Draht einen Verschlussankerteil und einen spiralförmigen Ankerteil bildet sowie einen geraden Verbindungsteil. In dem verschließenden Ankerteil nimmt der Draht die Form einer Reihe von Windungen an, die sich über den Querschnittsabschnitt des zu verschließenden Hohlraums erstrecken. Der gerade Verbindungsteil ist durch den Draht selbst als Verbindung zwischen den beiden Spiralen gebildet. Durch die jeweiligen Spiralen entsteht jedoch keine feste Tragstruktur, so dass ein Versagen des Implantats bzw. dessen ungewolltes Deplatzieren innerhalb der Defektöffnung nicht ausgeschlossen werden kann.

Aus der US 5.433.727 A ist ein Implantat zum Verschließen großer Defekte in großen Herzen bekannt, wie beispielsweise Atrium Septumdefekte (ASD) und Ventrikel Septumdefekte (VSD). Das Verschlussimplantat enthält eine entfaltbare Schaumharzscheibe, ein beschichtetes Drahtskelett in Form eines "X", aufgebracht auf die Schaumscheibe sowie eine einstellbare Schlaufe, angebracht an der Mitte des Drahtskeletts.

Im Stand der Technik sind außerdem noch sich knäuelförmig entfaltende Implantate bekannt, wie beispielsweise aus der US 4,994,069. Auch sich spiralförmig aufwickelnde Implantate sind bekannt, beispielsweise aus der DE 197 04 269 A1 oder als Röhrchen aus der WO 92/14408, wobei dieses Röhrchen ebenfalls spiralförmig oder helikal oder auch in Schlaufen gelegt aufgewickelt in einem Gefäß platziert wird. Ein weiteres spiralförmiges Implantat ist aus der WO 97/42881 bekannt. Ein gewendeltes Implantat ist aus der DE 32 03 410 bekannt. Weitere spiralförmige Implantate sind aus der DE 41 04 702 A1, EP 1 046 375 A1 und DD 158 084 bekannt. Bei all diesen spiralförmigen Implantaten ist keine feste Tragstruktur gegeben. Auch das Implantat gemäß der EP 0 378 151 A2 offenbart keine solche feste Tragstruktur. Bei diesem wird ein wellenförmig gebogener Draht zu miteinander verbundenen Schleifen gelegt. Eine feste Tragstruktur wird hingegen beispielsweise durch Verflechten von mehreren Drähten miteinander gebildet. In der Primär- oder Grundform entsteht dabei ein sog. Stent, der in der Sekundärform das Verhältnis von Länge zu Querausdehnung entlang einer Achse ändert, wobei dieses Verhältnis von Länge zu Querausdehnung kleiner wird als in der Primärform (Stent). WO 98/47430 offenbart eine Verschlusseinrichtung gemäß dem Oberbegriff des ersten Anspruchs.

Bei den vorstehend genannten Implantaten hat es sich als nachteilig erwiesen, dass sie entweder sehr kompliziert aufgebaut oder im menschlichen oder tierischen Körper zu implantieren sind oder dass sie keinen sicheren Halt innerhalb eines Gefäßes oder anderen Hohlraums im Körper des Menschen oder Tieres ermöglichen. Als besonders schwierig hat es sich hierbei auch herausgestellt, diese Implantate hinsichtlich ihrer Position zu verlagern, sofern sie während des Aussetzvorgangs fehlplatziert wurden. Insbesondere das Herausnehmen der Implantate aus dem Körper des Patienten ist durch deren Aufbau nur schwer möglich.

Der vorliegenden Erfindung liegt daher die Aufgabe zugrunde, diese Nachteile des Standes der Technik zu überwinden und insbesondere eine implantierbare Einrichtung vorzusehen, die sowohl für den Verschluss als auch für den Teilverschluss eines Organswegs, einer Defektöffnung, eines Hohlraums etc. sowie zum Schaffen einer definierten Verbindungsöffnung zwischen Wandungen, Organen, Hohlräumen etc. innerhalb eines menschlichen oder tierischen Körpers geeignet ist, also vielseitig variabel eingesetzt und auch wieder von dem Implantationsort entnommen werden kann, also implantierbar und extrahierbar ist.

Die Aufgabe wird durch eine implantierbare Einrichtung nach dem Oberbegriff des Anspruchs 1 dadurch gelöst, dass die Tragstruktur aus einem einzigen drahtartigen Element durch verschränkendes Wickeln und/oder Verwinden und/oder Verflechten nach Art eines Gewebes und/oder Geleges und/oder Netzes geformt ist, und durch die weiteren Merkmale des Anspruchs 1. Weiterbildungen der Erfindung sind in den abhängigen Ansprüchen definiert.

Dadurch wird eine implantierbare medizinische Einrichtung zum Gefäßverschluss bzw. Teilverschluss von Öffnungen und sonstigen Hohlräumen im menschlichen oder tierischen Körper geschaffen, die z.B. für die Verwendung bei VSD und ASD geeignet ist. Außerdem wird eine definierte Verbindungsöffnung zwischen Wandungen, Organen, Hohlräumen etc. innerhalb des menschlichen oder tierischen Körpers geschaffen. Die Einrichtung kann sehr vielfältig unter Verwendung lediglich eines drahtartigen Elementes ausgebildet werden. Insbesondere ist es möglich, im Bereich von Herzklappen Öffnungen zu verschließen, in welchem Bereich die derzeit bekannten Implantate nicht positioniert werden können aufgrund des dort herrschenden einseitigen Platzmangels. Die implantierbare Einrichtung kann aufgrund der Verwendung lediglich eines einzigen drahtartigen Elementes unsymmetrisch ausgebildet werden, insbesondere mit zweiseitig geöffneten Enden im Unterschied insbesondere zu dem vorstehend genannten Stand der Technik, in dem jeweils zumindest ein Ende der fertigen Implantate zusammengefasst ist. Dies beruht dort insbesondere darauf, dass ein Geflecht oder Gewebe aus zahlreichen drahtartigen Elementen verwendet wird. Im Unterschied zu den spiralförmigen implantierbaren Einrichtungen des Standes der Technik ist bei der erfindungsgemäßen eine Tragstruktur ausgebildet, die erheblich stabiler ist und außerdem die unterschiedlichsten Formen annehmen kann, insbesondere auch nichtsymmetrische, was bei den helikalen oder spiralförmigen implantierbaren Einrichtungen des Standes der Technik nicht möglich ist. Die Tragstruktur weist eine erheblich größere Widerstandsfähigkeit gegen von außen einwirkende Kräfte auf und kann dadurch sicherer als die spiralförmigen oder helikalen Einrichtungen des Standes der Technik im menschlichen oder tierischen Körper implantiert werden.

Die Tragstruktur wird durch verschränkendes Wickeln und/oder Verwinden des drahtartigen Elementes nach Art eines Gewebes. Geleges, Geflechts, Netzes oder dergleichen ausgebildet. Trotz der Verwendung nur eines drahtartigen Elements kann eine solche Tragstruktur durch Wickeln, Verschränken bzw. Verflechten gebildet werden, wobei die Tragstruktur eine Gewebe-. Gelege- und/oder Netzstruktur aufweist. Durch das verschränkende Wickeln und/oder Verwinden bzw. Verflechten des drahtartigen Elementes entsteht in der Primärform oder Grundwickelform im Wesentlichen ein schlauchförmiges Element. Dieses weist zwei offene Enden auf, wobei besonders bevorzugt die beiden Enden des drahtartigen Elementes an einem der Enden der Tragstruktur angeordnet oder in die Fläche der Tragstruktur integriert sind. Hierdurch ist zum einen ein möglichst geringes Verletzungsrisiko für den Patienten, bei dem die implantierbare Einrichtung implantiert wird, und zum anderen eine in sich feste Tragstruktur möglich. Dies ist mit den helikalen Implantaten des Standes der Technik nicht möglich, da deren Enden stets am äußeren Umfang von gewickelten Scheiben oder an den Enden der gewendelten Stents enden.

Wenn die beiden Enden des drahtartigen Elementes in die Fläche der Tragstruktur eingeflochten werden, sind diese gegen ein ungewolltes Lösen gesichert. Sofern die beiden Enden des drahtartigen Elementes an nur einem der Enden der Tragstruktur enden, kann das andere Ende mit einem sog. perfekten Rand versehen werden, also einem gleichmäßig ausgebildeten, im wesentlichen glatten Rand, so dass an diesem Ende vorteilhaft kein Verletzungsrisiko für das umgebende Gewebe der implantierbaren Einrichtung auftritt. Dasselbe kann auch bei Einflechten der Enden in die Tragstruktur oder deren Verbinden geschehen. Selbstverständlich ist es grundsätzlich auch möglich, dass die beiden Enden des drahtartigen Elementes an unterschiedlichen Enden oder an unterschiedlichen Positionen innerhalb der Tragstruktur und/oder an einem Ende herausragen oder verflochten sind.

Bevorzugt sind der proximale und der distale Abschnitt scheibenförmig mit einem dazwischen angeordneten Zwischenabschnitt ausgebildet, wobei der Zwischenabschnitt einen gegenüber dem proximalen und/oder distalen Abschnitt reduzierten Durchmesser aufweist. Durch diese Formgebung ist ein besonders guter Halt in einer Öffnung innerhalb einer Wandung im menschlichen oder tierischen Körper möglich, da sich der proximale und/oder der distale scheibenförmige Abschnitt auf beiden Seiten der Wandung sehr gut anlagern können. Je nachdem, wie der Zwischenabschnitt ausgebildet ist, kann dann entweder eine definierte Durchgangsöffnung durch diesen erzeugt werden oder auch ein vollständiger oder teilweiser Verschluss der Öffnung in der Wandung. Vorzugsweise wird aus dem nur einen drahtartigen Element durch entsprechendes Wickeln, Verschränken und Verdrehen zunächst ein langgestreckter Schlauch mit einer Gewebe-, Gelege- bzw. Netzstruktur gebildet, der nachfolgend im Bereich zwischen den beiden Enden des Schlauches hinsichtlich seines Durchmessers eingezogen und im Bereich seiner distalen und proximalen Enden herausgebogen wird, so dass dort jeweils ein größerer Durchmesser als im Zwischenabschnittsbereich entsteht. Nach dem Einprägen der Sekundärform, die beispielsweise einem schmalen Röhrchen mit zwei sehr großen Anlaufscheiben an den Enden ähnelt, kann ein im Wesentlichen flaches Gebilde entstehen. Die beiden scheibenförmigen proximalen und distalen Abschnitte sind in einer bevorzugten Ausführungsform im Wesentlichen flach, können in anderen Ausführungsformen jedoch auch konkav tellerförmig bzw. so weit zurückgebogen sein, dass ein einen Innenraum aufweisendes Gebilde entsteht. Zumindest einer der beiden proximalen und distalen Abschnitte ist in der Sekundärform bevorzugt in Richtung zu dem anderen zurückgebogen. Je nachdem, wie weit die beiden proximalen und distalen Abschnitte zueinander bzw. einer der beiden Abschnitte zu dem anderen zurückgebogen ist, entsteht eine mehr oder weniger ausgeprägte Pilz- oder Napfform, bevorzugt mit innerer Durchgangsöffnung. Vorzugsweise verbleibt in der Sekundärform der Tragstruktur eine mittlere Durchgangsöffnung in der implantierbaren Einrichtung zum Teilverschluss einer Öffnung im menschlichen oder tierischen Körper. Mittels eines solchen Teilverschlusses kann eine definierte Öffnung erzeugt und beispielsweise ein Hochdruck auf der einen Seite der implantierbaren Einrichtung in einen verminderten Druck auf der anderen Seite reduziert werden. Ein Anwendungsgebiet ist hierbei beispielsweise der Einsatz in einem zum Kopf führenden Blutgefäß, um in dem Blutgefäß herrschenden Bluthochdruck vor Erreichen des Kopfes, insbesondere im Schulterbereich, durch die implantierbare Einrichtung zu vermindern. Ein Einsatzbereich ist auch die Arteria Pulmonaris, in die eine solche implantierbare Einrichtung zum Teilverschluss eingesetzt werden kann. Neben einem Verschluss und einem Teilverschluss kann auch eine offene Verbindung zwischen zwei Wänden bzw. zwischen Organen geschaffen werden, zwischen denen eine vorbestimmte Öffnung verbleiben soll. Auch ein Einsatz in der Colostomie ist möglich.

Vorzugsweise ist eine innerhalb der implantierbaren Einrichtung vorgesehene Durchgangsöffnung exzentrisch in dieser angeordnet. Der proximale und der distale Abschnitt können zueinander versetzt sein, so dass der Zwischenabschnitt bzw. insbesondere die Durchgangsöffnung nicht zentral in der implantierbaren Einrichtung angeordnet ist, sondern dezentral. Gerade bei der Verwendung der implantierbaren Einrichtung zum Verschluss von Öffnungen im Bereich von Herzklappen erweist sich dies als vorteilhaft, wenn eine definierte Durchgangsöffnung bzw. der Zwischenabschnitt zwischen dem proximalen und dem distalen Abschnitt im Randbereich der implantierbaren Einrichtung angeordnet ist. Hierdurch kann trotz des einseitigen Platzmangels die Einrichtung fest und sicher implantiert werden. Die Anordnung der Durchgangsöffnung bzw. des Zwischenabschnitts kann je nach Anwendungsgebiet der Einrichtung beliebig gewählt werden.

Bevorzugt sind die Abmessungen und Form der implantierbaren Einrichtungen, einer Durchgangsöffnung innerhalb der implantierbaren Einrichtung und/oder des Randes der implantierbaren Einrichtung anwendungsfallbezogen wählbar oder einstellbar. Auch die Position der Durchgangsöffnung innerhalb der implantierbaren Einrichtung bzw. Tragstruktur ist anwendungsfallbezogen wählbar. Sie kann beispielsweise auch einseitig im Randbereich der Einrichtung ausgebildet werden. Besonders bevorzugt ist die Materialmenge im Randbereich der implantierbaren Einrichtung an die gewünschten Eigenschaften anpassbar, insbesondere ist eine Materialkonzentration im Randbereich der Einrichtung zur partiellen Versteifung vorgesehen. Wenn eine erfindungsgemäße implantierbare Einrichtung bevorzugt im Randbereich des proximalen und/oder distalen Abschnitts den größten Durchmesser aufweist, eignet sich dieser Bereich besonders für das gezielte Einstellen und Ändern der Stabilität der implantierbaren Einrichtung. Ist eine gleichmäßige Materialkonzentration über die Fläche der implantierbaren Einrichtung hinweg erzeugt, weist diese im Wesentlichen überall die gleiche Flexibilität und damit auch Verformbarkeit auf. Soll ein fester Halt beispielsweise innerhalb eines Blutgefäßes oder innerhalb einer sonstigen Röhre innerhalb des menschlichen oder tierischen Körpers erzeugt werden, kann der Randbereich der Tragstruktur besonders stabil durch dortige Materialkonzentration ausgebildet werden. Im Bereich der übrigen Fläche der Tragstruktur bleibt dann hingegen eine größere Flexibilität und Blegefähigkeit erhalten. Soll insbesondere innerhalb eines Blutgefäßes oder einer anderen Röhre innerhalb des menschlichen oder tierischen Körpers ein definierter Durchgang geschaffen werden, kann aus der schlauchförmigen Primär- oder Grundwickelform eine Ringform als Sekundärform gebildet werden, wobei insbesondere das gesamte Material nur im Randbereich konzentriert wird, so dass ein sehr stabiler Ring entsteht. Grundsätzlich kann in diesen hinein auch noch eine Membran gesetzt werden, so dass auch hierdurch ein Verschluss einer Öffnung möglich wird. Gerade aufgrund der Materialkonzentration im Randbereich kann dort ein sog. "perfekter Rand" gebildet werden. Dieser zeichnet sich dadurch aus, dass er im Wesentlichen eben ist, also dort keine Schlingen oder Schlaufen herausragen. Hierdurch ist auch ein besonders guter und wenig verletzungsintensiver Halt innerhalb von Röhren, insbesondere Blutgefäßen oder anderen Organwegen im menschlichen oder tierischen Körper möglich.

Bevorzugt ist zumindest ein Teilbereich der implantierbaren Einrichtung einfaltbar oder eingefaltet ausgebildet. Hierbei sind vorzugsweise der proximale und der distale Abschnitt der Tragstruktur in der Sekundärform flach und partiell so aufeinander gelegt, dass ein Verschluss oder Teilverschluss von seitlich durch Wandungen begrenzten Öffnungen, insbesondere im Bereich von Klappen, im menschlichen oder tierischen Körper möglich ist. In der Primär- oder Grundwickelform der implantierbaren Einrichtung ist diese vorzugsweise dabei wiederum schlauchförmig ausgebildet und lediglich in der Sekundärform entweder nur einseitig oder an mehreren Stellen eingefaltet, so dass beliebige Formgebungen möglich sind, wobei vorzugsweise die gesamte implantierbare Einrichtung bzw. Tragstruktur in der Sekundärform flach ausgebildet ist. Es können jedoch auch lediglich Teilbereiche, nämlich die aufeinandergefalteten, flach ausgebildet werden. Durch das Einfalten, insbesondere einseitige Einfalten eines Teils oder der gesamten Tragstruktur sind Formgebungen möglich, die auch in den ansonsten mit herkömmlichen Implantaten nicht zugänglichen Stellen positioniert werden können. Hierbei erweist es sich auch als besonders vorteilhaft, dass die gesamte implantierbare Einrichtung dabei im Wesentlichen flach sein kann.

Außerdem sind zumindest in einem Abschnitt der Tragstruktur in der Primär- und/oder Sekundärform nicht symmetrische und/oder unregelmäßige Ausbildungen möglich. Es muss also keine Rotationssymmetrie mehr vorhanden sein, wie es bei den implantierbaren Einrichtungen des Standes der Technik üblich und aufgrund des Aufbaus im Wesentlichen grundsätzlich nur möglich ist. Hierbei ist es besonders bevorzugt, dass die Materialkonzentration und/oder die Materialstärke innerhalb der Tragstruktur abschnittsweise unterschiedlich ist. Das drahtartige Element kann somit auch in sich unterschiedliche Material stärke, also einen unterschiedlichen Durchmesser aufweisen. Alternativ oder zusätzlich ist das Vorsehen von mehr als dem einen Draht zum Ausbilden der Tragstruktur möglich, wobei dieser Draht partiell aufgedoppelt bzw. verstärkt werden kann. Auch hierdurch können Materialkonzentrationen an bestimmten Stellen gezielt erreicht werden, so dass die Tragstruktur partiell steifer oder weniger steif ausgestaltet ist. Die Variantenvielfalt ist groß, da für einen jeweiligen Anwendungsfall eine bestimmte Form gezielt eingestellt und hinsichtlich ihrer Steifigkeit ebenfalls anwendungsspezifisch angepasst werden kann. Sofern distal- oder proximalseitig nur eine nicht symmetrische oder asymmetrische Struktur untergebracht werden kann, ist es auch möglich, den einen Abschnitt so auszubilden, dass in eine Richtung keine Erstreckung oder im Wesentlichen keine Erstreckung der Tragstruktur erreicht wird, wohingegen der andere Abschnitt in jede Richtung eine Erstreckung aufweist. Zumindest einseitig kann damit ein besonders guter Halt in einer Öffnung erzeugt werden, die zu der anderen Seite keinen Platz für ein gleichmäßig ausgebildetes Implantat aufweist.

Vorzugsweise ist das Ende des proximalen Abschnitts offen oder teilweise oder vollständig verschlossen, insbesondere durch Vorsehen eines Plattenelements. Nachdem die Tragstruktur aus lediglich einem drahtartigen Element gebildet wird, weist das Ende des proximalen Abschnitts vorzugsweise eine oder mehrere Schlingen oder Schlaufen auf, die nebeneinander und/oder miteinander verschränkt und/oder verschlungen angeordnet sind. Hierbei kann insbesondere auch ein im Wesentlichen gleichmäßiger Rand gebildet werden, wenn die Schlingen oder Schlaufen langgezogen miteinander verschränkt sind. Ein solcher Rand eignet sich gerade bei scheibenförmigen proximalen und distalen Abschnitten. Bildet der proximale Abschnitt beispielsweise einen kurzen Stutzen innerhalb der Fläche des distalen Abschnitts, weist der proximale Abschnitt an seinem Ende vorzugsweise Schlingen oder Schlaufen auf. Diese umgeben eine Durchgangsöffnung, wobei sie vorzugsweise als e-förmige Schlaufen nebeneinander oder als u-förmige Schlingen nebeneinander oder miteinander verschränkt zum exakten Umgrenzen der Durchgangsöffnung oder in der einen und/oder anderen Form ineinander verschränkt bzw. verschlungen angeordnet sein können, so dass in letzterem Fall die Durchgangsöffnung im Wesentlichen verschlossen wird. Ein vollständiger Verschluss kann insbesondere durch eine Befestigungsplatte als Plattenelement erfolgen. Diese kann dort auf gefügt, beispielsweise aufgeklebt, aufgenäht, aufgeschweißt etc., oder eingearbeitet sein, beispielsweise durch ein Durchfädeln mit den Schlingen am Ende des beispielsweise proximalen Abschnitts.

Vorzugsweise ist der distale und/oder proximale Abschnitt im Wesentlichen flach scheiben- oder ringförmig oder zumindest im Randbereich umgebogen oder zu einem distalen und proximalen verbindenden Zwischenabschnitt hin zurückgebogen, einen Innenraum umgrenzend ausgebildet. Je nachdem, wie weit der proximale oder distale Abschnitt umgebogen bzw. zurückgebogen ist, entsteht in der Seitenansicht ein kelchförmiges Gebilde oder ggf. sogar ein einseitig oder beidseitig (proximal und/oder distal) vollständig zurückgebogenes Gebilde, dessen Ende(n) in eine mittlere Durchgangsöffnung hineinragt/en. Durch den derart zurückgebogenen distalen und/oder proximalen Abschnitt wird jeweils dort ein Innenraum umgrenzt, wobei zugleich jedoch der Vorteil einer im Wesentlichen flachen Ausbildung erhalten bleibt. Selbstverständlich kann durch Auseinanderziehen des distalen und/oder proximalen Abschnitts auch eine Vergrößerung in Richtung der quer durch die implantierbare Einrichtung gehenden Achse hervorgerufen werden. Auch dies ist je nach Anwendungsfall beliebig variabel einstellbar.

Vorzugsweise ist die Tragstruktur als miteinander zu einem Teil verbundene zwei- oder mehrteilige Einheit aus einem drahtartigen Element gebildet. Sie kann jedoch ebenso aus einem durchgehend einteilig gewickelten Gebilde bestehen. Besonders bevorzugt sind die einzelnen Teile der Tragstruktur gleichmäßig, einander entsprechend oder unterschiedlich ausgebildet. Dadurch, dass bevorzugt nicht ein durchgehender Schlauch oder ein ähnliches Element gebildet wird, sondern verschiedene Teile, die aneinandergekettet sind, im Wesentlichen ohne eine Unterbrechung zwischen den einzelnen Teilen, kann eine größere Steifigkeit der Tragstruktur erzielt werden. Außerdem ist in dem Bereich des Aneinanderkettens der Teile eine besondere zusätzliche Aussteifung möglich. Außerdem können die einzelnen Teile unterschiedlich ausgebildet werden, wobei Lücken zwischen den einzelnen Teilen partiell möglich sind. Hierdurch können beim Umformen in die Sekundärform wiederum besondere Effekte erzielt werden, insbesondere unsymmetrische oder asymmetrische Sekundärformen erzeugt werden. Außerdem können Tragstrukturen mit über den Querschnitt verteilter unterschiedlicher Steifigkeit erstellt werden. Würde eine solche in der Primär- oder Grundwickelform stentartige Tragstruktur mit distalseitigen Schlaufen und Öffnungen bzw. Lücken in der Struktur ausgebildet, könnte diese auch zum Einfangen von Objekten in Organwegen oder Körperorganen oder Öffnungen verwendet werden. Die einzelnen Abschnitte der Grundwickelform können auch unterschiedliche Wickelwinkel aufweisen, wodurch ebenfalls unterschiedlich steife Bereiche entstehen können.

Vorzugsweise sind eine oder mehrere Membranen oder membranartige oder membranbildende Strukturen in die Tragstruktur eingebracht oder auf diese aufgebracht. Bevorzugt ist die membranbildende Struktur durch Einflechten zumindest eines Fadens gebildet, insbesondere eines Fadens aus einem flexiblen flechtbaren Material, insbesondere einem Kunststoff, nachwachsenden Rohstoff oder Metall. Besonders vorteilhaft können Dacronfäden in eine aus Nitinol gebildete Tragstruktur eingeflochten und/oder ein Gewebe. Gelege. Geflecht oder dergleichen aus diesen Fäden gebildet und in die Tragstruktur eingebracht werden. Alternativ oder zusätzlich eignen sich auch Carbonfasern. Mittels dieser Fasern bzw. Fäden kann eine durch die implantierbare Einrichtung hindurchgehende Durchgangsöffnung verschlossen werden. Durch das Vorsehen von Membranen, membranartigen oder membranbildenden Strukturen in der Tragstruktur kann ein vollständiger Verschluss von beispielsweise Defektöffnungen im Körper eines Menschen oder Tieres erzeugt werden. Je nach Ausbildung und Anordnung der Membran innerhalb der Tragstruktur kann auch ein Teilverschluss hierdurch geschaffen werden. Wie bereits weiter oben erwähnt, kann auch in eine ringförmige Sekundärform der Tragstruktur eine solche Membran eingebracht werden oder eine membranbildende Struktur, so dass je nach Flexibilität der Membran oder membranbildenden Struktur ein sich in gewissem Maße flexibel bewegendes, jedoch fest in einem Organweg eingebrachtes Gebilde entsteht. Derartige einbringbare Membranen können vorgefertigte textile Gelege. Gewebe. Geflechte oder dergleichen sein und sogar in das zur Tragstruktur verwobene bzw. verschränkt gelegte drahtartige Element der Tragstruktur integriert werden. Auch eine Kombination von beiden Möglichkeiten ist möglich. Beispielsweise ist ein nachträgliches Einflechten zumindest eines Fadens zum Ausbilden einer Membranstruktur möglich. Der Faden oder das Material der membranbildenden Struktur unterscheidet sich vorzugsweise von dem des drahtartigen Elementes, kann jedoch auch den gleichen Querschnitt aufweisen. Vorzugsweise besteht die membranbildende Struktur aus einem Material mit einem dünneren Querschnitt oder weist ein Geflecht, Gelege, Gewebe mit Fäden unterschiedlichen Durchmessers auf. Um unterschiedliche Dichtigkeiten innerhalb der membranbildenden Struktur zu schaffen, weist der Faden bzw. das Material der membranbildenden Struktur vorzugsweise in sich unterschiedliche Durchmesser auf oder es sind mehrere parallel geführte Fäden vorgesehen. Die membranartige Struktur kann bevorzugt alternativ auch durch Tauchen der Tragstruktur in ein filmbildendes Material gebildet werden. Ein solches Material kann insbesondere ein aus einem oder mehreren Monomeren gebildetes natürliches oder synthetisches Polymer sein, insbesondere gebildet durch Polyaddition, Polymerisation oder Polykondensation, insbesondere ein Polycarbonat, Polyester, Polyamid, Polyolefin oder Polyurethan. Auch Polystyrole eignen sich. Je nach Anwendungsfall wird vorzugsweise ein Material gewählt, das eine größere oder geringere Flexibilität bzw. Oberflächenspannung aufweist und insbesondere im menschlichen und tierischen Körper nicht Abstoßungsprozesse auslöst. Abhängig davon, ob sich auf der membranartigen Struktur etwas, z.B. Gewebe anlagern soll, kann die Materialwahl in Richtung hydrophober oder hydrophiler Materialien gehen. Auch eine Beschichtung der membranartigen Struktur und/oder von Fäden einer Membran mit solchen Materialien ist möglich.

Vorzugsweise ist die membranartige Struktur oder Membran aus einem Gewebe. Gelege oder anderen Textil gebildet und im Randbereich mit auskragenden Armen zum Einfädeln und/oder Befestigen an der Tragstruktur versehen, insbesondere durch Vernähen, Verkleben. Verschweißen, Crimpen oder ein anderes Befestigungsverfahren. Hierbei werden insbesondere die auskragenden Arme um den Randbereich der Tragstruktur am distalen und/oder proximalen Ende herumgeführt und auf der Oberfläche der membranartigen Struktur oder Membran aufgefügt und dort befestigt. Hierdurch ist ein besonders guter und verrutschsicherer Halt auf der Tragstruktur möglich.

Die Membran oder Membranen, membranartige(n) oder membranbildende(n) Struktur(en) ist/sind vorzugsweise proximal und/oder distal und/oder im Wesentlichen zentral in der Tragstruktur angeordnet. Die Membran(en), membranartige(n) oder membranbildende(n) Struktur(en) kann/können auch schräg innerhalb der Tragstruktur angeordnet werden, also z.B. von der proximalen zur distalen Seite wenn dies auf den Anwendungsfall bezogen zweckdienlich erscheint. Es ist auch eine lediglich partielle Anordnung von diesen innerhalb der Tragstruktur möglich, insbesondere um einen Teilverschluss zu ermöglichen.

Vorzugsweise ist das Material der Tragstruktur in zumindest einem Teilbereich chemisch und/oder mechanisch behandelt, insbesondere geätzt, elektropoliert, mikrogeschliffen oder anderweitig behandelt. Hierdurch können unterschiedliche Materialstärken und dadurch unterschiedliche Steifigkeiten erzeugt werden. Eine solche Behandlung eignet sich insbesondere bei einem geschnittenen Rohr. Besonders bevorzugt besteht das drahtartige Element bzw. das geschnittene Rohr der implantierbaren Einrichtung aus einem biokompatiblen Material, insbesondere einem Metall oder einer Metalllegierung, insbesondere einem Edelstahl oder Formgedächtnismaterial, wie Nitinol oder einem Kunststoff, wie beispielsweise Polycarbonat. Es wird bevorzugt ein solches Material verwendet, mit dem es möglich ist, unterschiedliche Primär- und Sekundärformen auszubilden, wobei diese so in das Material eingeprägt werden, dass beim Platzieren der implantierbaren Einrichtung innerhalb eines menschlichen oder tierischen Körpers automatisch die Sekundärform angenommen wird bzw. ein beliebiger Wechsel zwischen der Primärform und der Sekundärform, insbesondere unter Verwendung von weiteren Hilfsmitteln möglich ist. Um eine Verträglichkeit mit dem tierischen und/oder menschlichen Körper zu ermöglichen, wird insbesondere ein biokompatibles Material verwendet. Aus Polycarbonaten können Strukturen gebildet werden, insbesondere durch Laserschneiden, die gewebe-, gelege- oder netzähnlich sind. Durch Aufbringen eines geeigneten Filmmaterials kann eine Membran, wie oben beschrieben, auch an einer solchen Tragstruktur vorgesehen werden. Durch einen Laserschnitt ist eine höhere Flexibilität als bei Verschränken bzw. Verwinden eines drahtartigen Elements möglich. Besonders eine aus einem Polycarbonat gebildete Tragstruktur weist ebenfalls eine gute Faltbarkeit auf. Hierdurch ist der Transport zu der Implantationsstelle besonders problemlos möglich. Dasselbe gilt für Carbonfasern. Aus diesen kann die Tragstruktur erstellt werden, insbesondere auch durch Nähen. Durch Eintauchen der erstellten Tragstruktur in Polyurethan kann ebenfalls eine Formgedächtnisstruktur gebildet werden. Durch Verwendung von Carbonfasern bzw. Polycarbonaten kann die Verwendung von Metallen entfallen, die teilweise von den Patienten nicht so gut vertragen werden.

Eine Detektion der Position der implantierbaren Einrichtung während eines Implantationsvorgangs kann vorteilhaft durch Röntgen oder durch eine 3D-Echographie erfolgen. Bei Verwendung eines Metallmaterials für die Tragstruktur der implantierbaren Einrichtung kann vorteilhaft eine Röntgendetektion vorgenommen werden, wohingegen sich bei der Verwendung eines Polycarbonats zum Ausbilden der Tragstruktur und/oder von Dacronfäden und/oder Carbonfasern zum Ausbilden einer Membran oder membranartigen Struktur zur Detektion eine 3D-Echographie eignet.

Zum Platzieren der implantierbaren Einrichtung innerhalb des menschlichen oder tierischen Körpers ist vorzugsweise ein Platziersystem vorgesehen. Bei diesem sind bevorzugt der oder die Haltedrähte durch eine oder mehrere Schlaufe(n) oder Schlinge(n) am Ende des proximalen Abschnitts fädelbar oder gefädelt und mit dem Führungsdraht und/oder Innenmandrin verbindbar. Auch ein Anbringen der Haltedrähte am distalen Ende ist möglich.

Insbesondere kann ein Haltedraht durch alle Schlaufen oder Schlingen am Ende des proximalen Abschnitts gefädelt werden. In einer alternativen Ausführungsform können beispielsweise zwei Haltedrähte durch alle oder mehrere Schlaufen oder Schlingen am Ende des proximalen Abschnitts, die in einer Hälfte davon angeordnet sind, durchgefädelt werden, wobei der Führungsdraht oder Innenmandrin vorzugsweise durch eine Schlaufe zwischen den beiden Haltedrähten, die von diesen gebildet wird, ragt, so dass die Haltedrähte dort einen Befestigungspunkt haben. Bei Zurückziehen des Führungsdrahtes bzw. Innenmandrins aus dieser Schlaufe heraus können nachfolgend auch die Haltedrähte zurückgezogen werden, so dass sich der proximale Abschnitt in diesem Bereich vollständig entfalten kann. Alternativ können auch mehrere Haltedrähte durch mehrere Schlingen oder Schlaufen am Ende des proximalen Abschnitts gefädelt werden, wobei die Haltedrähte vorzugsweise immer nur durch einen Teil der Schlingen oder Schlaufen gefädelt werden. Es kann hierbei eine beliebige Anzahl von Haltedrähten vorgesehen werden, insbesondere jeweils auch ein Haltedraht je Schlaufe oder Schlinge, wobei bevorzugt eine Kette aus Haltedrahtschlaufen gebildet wird und der Führungsdraht oder Innenmandrin bevorzugt nur durch eine Schlaufe am Ende der Kette aus Drahtschlaufen gezogen wird.

Sind alle Schlaufen oder Schlingen am Ende des proximalen Abschnitts zusammengeführt, kann es ausreichen, lediglich einen Haltedraht mit einer Schlaufe und einen Führungsdraht oder Innenmandrin vorzusehen, der durch diese Schlaufe geführt wird. Beispielsweise können 1, 2, 10, 12. 18, 24 oder eine beliebig andere Anzahl von Schlaufen oder Schlingen am Ende der jeweiligen proximalen und distalen Abschnitte gebildet sein, durch die eine beliebige Anzahl von Haltedrähten gefädelt werden kann.

Zum Platzieren der implantierbaren Einrichtung an einem Ort innerhalb des menschlichen oder tierischen Körpers wird vorzugsweise ein Katheter sowie ein Vorschubröhrchen, insbesondere ein Führungsdraht und ein Haltedraht bzw. Haltedrähte verwendet. Zunächst werden dabei die Schlaufen oder Schlingen am Ende des proximalen Abschnitts und insbesondere auch des distalen Abschnitts mittels des einen oder mehrerer Haltedrähte aufgefädelt und der Führungsdraht so positioniert, dass der Haltedraht mit in diesem filiert wird, so dass ein vorzeitiges Lösen von der implantierbaren Einrichtung dadurch im Wesentlichen vermieden wird. Nachfolgend wird das Vorschubröhrchen über Führungsdraht und Haltedrähte hinweg positioniert und die implantierbare Einrichtung in das Vorschubröhrchen hineingezogen, wobei es eine langgestreckte Form annimmt. Der Katheter wird in den Körper des Patienten eingeführt, wobei das Vorschubröhrchen vorzugsweise bereits in den Katheter eingeführt ist oder nachfolgend noch wird. Der Katheter wird so weit vorgeschoben, dass er im Bereich der Stelle positioniert ist, an der die implantierbare Einrichtung ausgesetzt werden soll. Nachfolgend wird zunächst das Vorschubröhrchen und anschlieBend der Führungsdraht mit der implantierbaren Einrichtung zusammen aus dem Katheter und nachfolgend aus dem Vorschubröhrchen herausgeschoben und an der Stelle, an der es positioniert werden soll, ausgesetzt.

Mit dem Platziersystem ist es ebenfalls möglich, eine implantierbare Einrichtung auch wieder aus dem Körper des Patienten zu extrahieren. Dies kann entweder nach einem Fehlpositionieren oder der erfolgten Heilung sinnvoll bzw. erforderlich werden. Bei dem erfindungsgemäßen Platziersystem ist zu diesem Zweck des Extrahierens der implantierbaren Einrichtung aus dem Implantationsort im menschlichen oder tierischen Körper bevorzugt ein Führungsdraht und ein Extraktionsdraht vorgesehen, wobei der Extraktionsdraht zu einer Schlinge legbar und durch zumindest eine Schlinge oder Schlaufe an einem Ende der Tragstruktur fädelbar ist. Für den Extraktionsvorgang wird vorzugsweise zunächst zumindest eine Schlinge des zumindest einen Extraktionsdrahtes durch die implantierbare Einrichtung hindurchgeführt. Der Führungsdraht wird ebenfalls durch die implantierbare Einrichtung hindurchgeführt sowie durch die Schlinge des Extraktionsdrahtes. Durch Ziehen an dem Extraktionsdraht und an dem Führungsdraht hält sich die Schlinge an dem Führungsdraht fest, jedenfalls, wenn Führungsdraht und Extraktionsdraht nicht denselben Weg durch die implantierbare Einrichtung genommen haben. Durch Zug an beiden Drähten kann die implantierbare Einrichtung in einen Katheter hineingezogen und ggf, von dem Implantationsort vollständig entfernt werden. Auch ein Umpositionieren ist auf diese Art und Weise möglich.

Es wird vorzugsweise ein Set aus mehreren unterschiedlich geformten implantierbaren Einrichtungen zusammen mit zumindest einem Haltedraht und/oder Extraktionsdraht, wobei beide gleich ausgeführt sein können, einem Führungsdraht und/oder Innenmandrin und ggf, einem Katheter angeboten. Insbesondere können Katheter und Haltedraht bzw. Extraktionsdraht sowie Führungsdraht und/oder Innenmandrin auch mehrfach verwendet werden, wohingegen die implantierbare Einrichtung üblicherweise in dem Patienten verbleibt.

Alternativ oder zusätzlich zu dem vorstehend beschriebenen Platziersystem kann ein solches ein Vorschubelement, eine in einem ersten Betriebszustand (Primärform) ein großes Verhältnis von Länge zu Querausdehnung entlang einer Achse und in einem zweiten Betriebszustand (Sekundärform) ein kleineres Verhältnis von Länge zu Querausdehnung entlang der Achse aufweisenden Hilfsstruktur zum Unterstützen des Entfaltens des proximalen Endes der Tragstruktur und zumindest eine Verbindungseinrichtung zum Verbinden des proximalen Endes der implantierbaren Einrichtung und des distalen Endes der Hilfsstruktur aufweisen. Sofern sich beim Entfalten des proximalen Endes der Tragstruktur Probleme ergeben, kann das Vorsehen einer solchen Hilfsstruktur Abhilfe schaffen. Die Hilfsstruktur wird an ihrem distalen Ende mit dem proximalen Ende der Tragstruktur verbunden und mit dieser zusammen über z.B. einen Katheter zu der Implantationsstelle gebracht. Nach dem Entfalten des distalen Endes der Tragstruktur werden das proximale Ende der Tragstruktur und das mit diesem verbundene distale Ende der Hilfsstruktur entfaltet. Das distale Ende der Hilfsstruktur ist so ausgebildet, dass es beim eigenen Entfalten das proximale Ende der Tragstruktur mit entfaltet. Durch Verwendung eines solchen Platziersystems kann die Tragstruktur genau und so positioniert werden, dass nach deren Abwerfen im Wesentlichen keine Bewegung mehr zugelassen wird. Bevorzugt weist die Verbindungseinrichtung zumindest einen Haltedraht, insbesondere drei Haltsdrähte, auf. Zum Verbinden der beiden Strukturen ist bereits die Verwendung eines Haltedrahtes ausreichend. Bei Verwendung dreier Haltedrähte ist ein nahezu kraftloses Aufspannen und Entfernen möglich. Vorzugsweise ist der zumindest eine Haltedraht durch eine oder mehrere Schlaufe(n) oder Schlinge(n) am Ende des proximalen Endes der implantierbaren Einrichtung und des distalen Endes der Hilfsstruktur fädelbar oder gefädelt. Hierdurch ist auf dem Weg zur Implantationsstelle eine gute Verbindung zwischen den Strukturen und ein leichtes Entfernen des zumindest einen Haltedrahts nach dem Aufspannen des proximalen Endes der Tragstruktur möglich.

Vorzugsweise werden die Enden des drahtartigen Elementes miteinander geeignet verbunden, um die Verletzungsgefahr für den Patienten zu verringern. Besonders bevorzugt wird eine Hülse oder ein rohrförmiges Element auf die Enden aufgefügt und dort insbesondere durch Verpressen oder Verkleben befestigt. Außerdem kann ein anderes Element, wie eine Spirale oder ähnliches auf die beiden Enden aufgefügt werden. Auch ein direktes Verbinden, insbesondere durch Schweißen, Löten. Verdrehen oder Verkleben oder dgl. ist möglich.

Die erfindungsgemäßen implantierbaren Einrichtungen können also verschiedene Funktionen erfüllen. Sie können zum einen durch einen Katheter in den Körper des Patienten eingeführt werden, dem Verschluss. Teilverschluss oder dem Vorsehen einer definierten Durchgangsöffnung dienen. Sie sind nicht störend für andere Strukturen innerhalb des Körpers des Patienten, sind implantierbar und halten sich selbständig nach dem Absetzen an dem Implantationsort fest, können jedoch auch, wenn sie störend wirken oder eine Fehlpositionierung stattgefunden hat, wieder von der Implantationsstelle entfernt werden. Bislang wurden Implantate mit einem Traggerüst versehen, um stabil zu sein. Die erfindungsgemäßen implantierbaren Einrichtungen weisen bereits durch ihre Formgebung eins ausreichende Stabilität auf und können sich durch diese auch selbständig in die gewünschte Position bringen. Sie sind nicht mehr zwingend symmetrisch ausgebildet und können daher gezielt entsprechend der Implantationsstelle ausgebildet werden und sich dort selbständig in richtiger Ausrichtung positionieren.

Zur näheren Erläuterung der Erfindung werden im Folgenden Ausführungsbeispiele anhand von Zeichnungen näher beschrieben. Diese zeigen in:
- Figur 1, 1a: eine Draufsicht und seitliche Draufsicht auf eine erste Ausführungsform einer implantierbaren Einrichtung.
- Figur 2: eine Draufsicht auf eine zweite Ausführungsform einer implantierbaren Einrichtungen.
- Figuren 2a bis 2d: Schnittansichten verschiedener weiterer Ausführungsformen einer Abwandlung der Ausführungsform gemäß Figur 2.
- Figur 3, 3a: eine Draufsicht und Seitenansicht auf eine weitere Ausführungsform einer implantierbaren Einrichtung, bei der eine Durchgangsöffnung unsymmetrisch angeordnet ist.
- Figur 4, 4a: eine Draufsicht und Seitenansicht auf eine weitere Ausführungsform einer implantierbaren Einrichtung, die einen eingefalteten Teilbereich aufweist,
- Figur 5: eine Draufsicht auf eine weitere Ausführungsform einer implantierbaren Einrichtung, die einen unregelmäßig ausgebildeten distalen Abschnitt aufweist,
- Figur 6: eine Draufsicht auf eine weitere Ausführungsform einer implantierbaren Einrichtung mit einem Randbereich des distalen Abschnitts mit Materialkonzentration und einem proximalen Abschnitt, der sehr kurz ausgebildet ist und nebeneinanderliegende Schlaufen am Ende aufweist,
- Figuren 6a bis 6d: Detailansichten weiterer Varianten für die Ausbildung des proximalen Abschnitts der implantierbaren Einrichtung nach Figur 6.
- Figur 7: eine Draufsicht auf eine weitere Ausführungsform einer erfindungsgemäßen implantierbaren Einrichtung mit zu dem Zwischenabschnitt zurückgebogenen proximalen Abschnitt,
- Figuren 7a bis 7c: weitere Varianten der Ausführungsform gemäß der erfindungsgemäßen implantierbaren Einrichtung gemäß Figur 7,
- Figur 8a bis 8e: Seitenansichten von Ausführungsvarianten von Grundwickelformen einer erfindungsgemäßen implantierbaren Einrichtung, die in zwei zusammenhängenden Teilen aus lediglich einem drahtartigen Element ausgebildet ist,
- Figur 9: eine weitere Ausführungsform einer implantierbaren Einrichtung, bei der diese aus mehreren Teilen an- bzw. ineinander zusammengesetzt ist, wobei lediglich ein drahtartiges Element verwendet ist,
- Figur 10a bis 10c: schematische Ansichten verschiedener Ausbildungen des Randbereichs eines distalen Abschnitts,
- Figur 11a bis 11e: Draufsichten auf verschiedene Ausführungsformen von implantierbaren Einrichtungen, die mit Membranen bzw. membranartigen oder membranbildenden Strukturen versehen sind,
- Figur 12: eine Draufsicht auf eine weitere Ausführungsform einer Membran für eine erfindungsgemäße implantierbare Einrichtung.
- Figur 12a, 12b: Draufsichten auf in Tragstrukturen eingebrachte Membranen nach Figur 12.
- Figur 13a bis 13g: Ablauf des Platzierens einer implantierbaren Einrichtung.
- Figur 14a bis 14c: Detailansichten von Enden proximaler Abschnitte von implantierbaren Einrichtungen, bei denen Haltedrähte und Führungsdrähte eines erfindungsgemäßen Platziersystems angeordnet sind,
- Figur 15: eine Detailansicht eines Endes einer implantierbaren Einrichtung mit Extraktionsdraht und Führungsdraht,
- Figur 16 bis 16c: Draufsichten auf Ausführungsformen von Endbefestigungen der Enden eines drahtartigen Elements.
- Figur 17a bis 17e: eine schematische Darstellung des Ablaufs der Herstellens einer implantierbaren Einrichtung.
- Figur 18a bis 18j: eine schematische Darstellung des Ablaufs einer alternativen Ausführungsform eines Platzierens einer implantierbaren Einrichtung,
- Figur 19a und 19b: perspektivische Ansichten von zwei Schritten des Platzierens mittels des Platziersystems nach Figur 18a bis 18j, und
- Figur 20: eine schematische Ansicht des Durchfädelns von Haltedrähten bei dem Platziersystem nach Figur 19a und 19b.

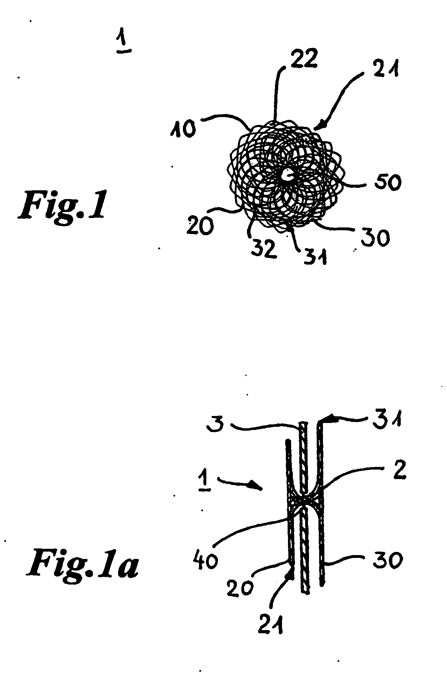

Figur 1 zeigt eine Draufsicht auf eine erste Ausführungsform einer implantierbaren Einrichtung 1. Diese ist aus zwei scheibenförmigen proximalen und distalen Abschnitten 20, 30 und einem dazwischen angeordneten Zwischenabschnitt 40 gebildet. Innerhalb des Zwischenabschnitts ist eine Durchgangsöffnung 50 vorgesehen. Wie aus der Figur 1a zu entnehmen, welche eine seitliche Draufsicht auf die Ausführungsform gemäß Figur 1 darstellt, sind die beiden proximalen und distalen Abschnitte 20. 30 jeweils nicht symmetrisch zu dem Zwischenabschnitt angeordnet, sondern zueinander und zu diesem versetzt. Die Durchgangsöffnung 50 ist somit nicht zentral in den beiden proximalen und distalen Abschnitten angeordnet. Der Zwischenabschnitt 40 ist dadurch gebildet, dass aus einer schlauchförmigen Primärform der implantierbaren Einrichtung 1 durch Verwinden bzw. Verdrehen zwei Teile abgeteilt werden, nämlich der proximale und der distale Abschnitt 20. 30. Nachfolgend werden die proximalen und distalen Enden dieser Abschnitte nach außen herausgezogen bzw. gebogen, bis die scheibenförmigen Abschnitte entstehen. Im äußeren Randbereich 21, 31 des distalen und proximalen Abschnitts sind aufgrund der Verwendung lediglich eines drahtartigen Elementes 10 für die gesamte implantierbare Einrichtung Schlaufen 22, 32 gebildet. Diese Schlaufen können auch zum Durchfädeln von Haltedrähten während des Platzierens an dem Ort innerhalb eines menschlichen oder tierischen Körpers dienen, an dem die implantierbare Einrichtung ihre Funktion erfüllen soll, nämlich einen vollständigen oder teilweisen Verschluss von Defektöffnungen, Hohlräumen, Organwegen etc. oder eine definierte Verbindungsöffnung zwischen Wandungen, Organen, Hohlräumen etc. zu bilden. Wie in Figur 1a angedeutet, positioniert und entfaltet sich die implantierbare Einrichtung 1 nach dem Aussetzen in einer solchen Defektöffnung 2 beispielsweise in einer Wandung 3 im Herzen eines Menschen.

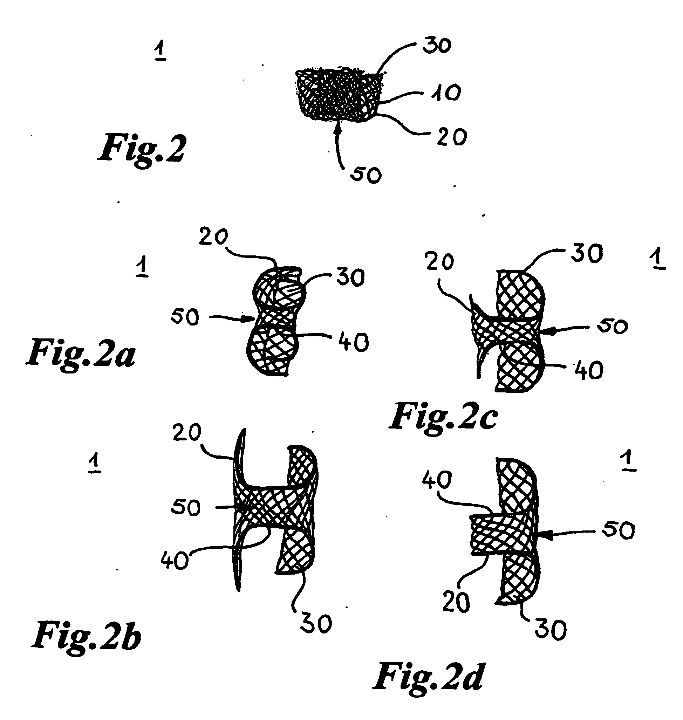

Die in den Figuren 2 bzw. 2a bis 2d dargestellten Ausführungsformen einer implantierbaren Einrichtung 1 unterscheiden sich von der in Figur 1 dargestellten insbesondere dadurch, dass sie nicht so flach bauen und außerdem besonders gut zur Druckverminderung in Gefäße implantiert werden können. Die in diesen Figuren dargestellten Ausführungsformen sind ebenfalls alle aus nur einem drahtartigen Element 10 gebildet. In der in Figur 2 dargestellten Ausführungsform ist der proximale Abschnitt 20 in Richtung zu dem distalen 30 zurückgebogen, so dass eine Kranzform mit innerer Durchgangsöffnung 50 entsteht. Der distale Abschnitt ist dabei im Wesentlichen zylindrisch. Auch die zweite Hälfte des proximalen Abschnitts läuft im Wesentlichen zylindrisch aus. Beim Implantieren in ein Gefäß oder ein anderes schlauch- oder röhrenförmiges Organ wird lediglich ein Teilverschluss erzeugt, wobei durch die Durchgangsöffnung 50 weiterhin ein Durchtritt von beispielsweise Blut oder einer anderen Körperflüssigkeit möglich ist.

Bei der Ausführungsform gemäß Figur 2a sind sowohl der distale Abschnitt 30 als auch der proximale Abschnitt 20 aufeinander zu gebogen. Hierdurch entsteht ein flacheres Gebilde als bei der Figur 2. Wie weit der distale und der proximale Abschnitt aufeinander zu gebogen sind, kann anwendungsspezifisch beliebig gewählt werden. Aufgrund der beiden aufeinander zu gebogenen Enden des distalen und des proximalen Abschnitts wird eine noch bessere Federwirkung in Querrichtung in diesem Bereich und dadurch beim Einbringen in ein Gefäß ein noch besserer Halt geschaffen als dies mit der Ausführungsform beispielsweise nach Figur 2 möglich ist. Die Ausführungsform nach Figur 2b weist einen im Wesentlichen flachen proximalen Abschnitt 20 und einen auf diesen zugebogenen distalen Abschnitt 30 auf. Wiederum verbleibt zwischen beiden die Durchgangsöffnung 50, wobei der Zwischenabschnitt 40 im Unterschied zu der Figur 2a länger und zylindrischer ist. In dieser Ausführungsform überragt hinsichtlich der seitlichen Umfangserstreckung der proximale Abschnitt den distalen bzw. den durch das Ende des distalen Abschnitts gebildeten Umfang.

In der in Figur 2c dargestellten Ausführungsform ist der distale Abschnitt wiederum zu dem proximalen hin umgebogen, jedoch ist der Zwischenabschnitt 40 kürzer als in der Ausführungsform nach Figur 2b und auch der proximale Abschnitt ist nicht im Wesentlichen scheibenförmig nach außen gebogen, sondern lediglich geringfügig gebogen, so dass er einen kleineren Umfang und Durchmesser beschreibt als das Ende des distalen Abschnitts. Die in der Figur 2d dargestellte Ausführungsform stellt im Wesentlichen eine Umkehr der in Figur 2 dargestellten Ausführungsform dar, da in dieser Ausführungsform nach Figur 2d der distale Abschnitt 30 zu dem proximalen 20 zurückgebogen ist, der proximale Abschnitt jedoch im Wesentlichen zylindrisch bleibt.

Aus den Figuren 2 bzw. 2a bis 2d ergibt sich, dass zahlreiche Varianten möglich sind, bei denen jeweils der distale und/oder proximale Abschnitt zu dem jeweils anderen zurückgebogen ist, wobei eine innere Durchgangsöffnung 50 zwischen den Abschnitten verbleibt. Somit ist insbesondere ein Teilverschluss bzw. eine offene Verbindung mit definiertem Durchmesser zwischen zwei Wänden, oder innerhalb eines Gefäßes möglich. Bei all den in den Figuren 2 bis 2d dargestellten Ausführungsformen wird jeweils eine besondere Kraft für die Befestigung der Einrichtung 1 im Körper des Patienten zur Verfügung gestellt, die zugleich ausreichend elastisch ist, so dass sich bewegende Organwege oder Gefäße mit einer solchen implantierbaren Einrichtung 1 versehen werden können, ohne dass die Gefahr besteht, dass diese sich ungewollt innerhalb des Gefäßes oder Organweges bewegt. Vielmehr hält sich die implantierbare Einrichtung 1 darin besonders gut fest.

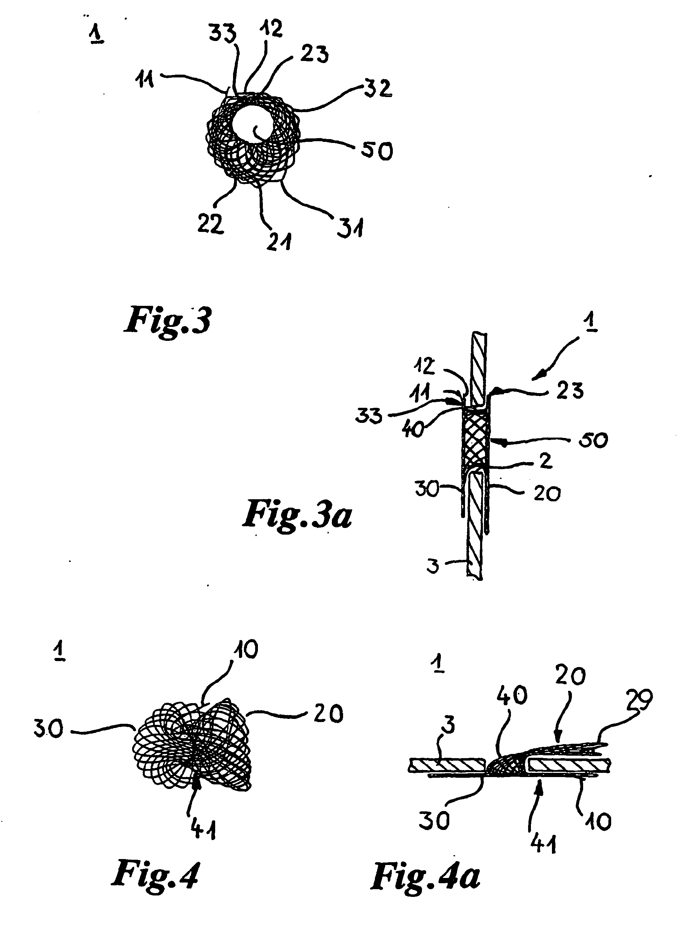

Die Figuren 3 und 3a zeigen eine weitere Ausführungsform einer implantierbaren Einrichtung 1. Figur 3 zeigt dabei eine Draufsicht und Figur 3a eine Seitenansicht der Einrichtung 1 in einem in eine Wandung eingefügten Zustand. Die Durchgangsöffnung 50 ist exzentrisch im Randbereich 21, 31 des proximalen und des distalen Abschnitts angeordnet. Proximaler und distaler Abschnitt liegen im Wesentlichen flach und mehr oder weniger deckungsgleich aufeinander. Der Randbereich 23, 33, der der Durchgangsöffnung 50 seitens des proximalen und des distalen Abschnitts direkt benachbart ist, ist mit einer größeren Materialkonzentration gefertigt. Dies bedeutet, dass in diesem Bereich die Maschenweite der durch das drahtartige Element gebildeten Schlaufen bzw. Maschen geringer ist, so dass diese Randbereiche 23, 33 steifer sind als die übrigen Randbereiche 21, 31 des distalen und des proximalen Abschnitts.

Aus Figur 3 ist ersichtlich, dass die Durchgangsöffnung an einer beliebigen Stelle in der implantierbaren Einrichtung angeordnet werden kann, dass sie auch einen beliebig variablen Durchmesser aufweisen kann, dass auch die Materialkonzentration sich über den Querschnitt der implantierbaren Einrichtung hinweg, insbesondere des proximalen und des distalen Abschnitts hinweg, ändern kann, insbesondere im Randbereich, und dass die Maschenweite sowie Umfang und Größe des proximalen und des distalen Abschnitts beliebig variabel an den Anwendungsfall angepasst gewählt werden können. Insbesondere ist im Vergleich zu Figur 1 ersichtlich, dass der Durchmesser der Durchgangsöffnung 50 in der Figur 3 größer ist, so dass ein Teilverschluss bei Einsatz in einer Defektöffnung oder dergleichen im menschlichen oder tierischen Körper gebildet wird.

Wie weiter unten noch erläutert werden wird, kann jedoch jede der in den vorigen und in den folgenden Figuren dargestellten Durchgangsöffnungen mit einer Membran oder einem membranartigen oder membranbildenden Element bzw. einer solchen Struktur mehr oder weniger verschlossen werden. Hierdurch wird dann auch ein vollständiger Verschluss möglich, trotz großer Durchgangsöffnung.

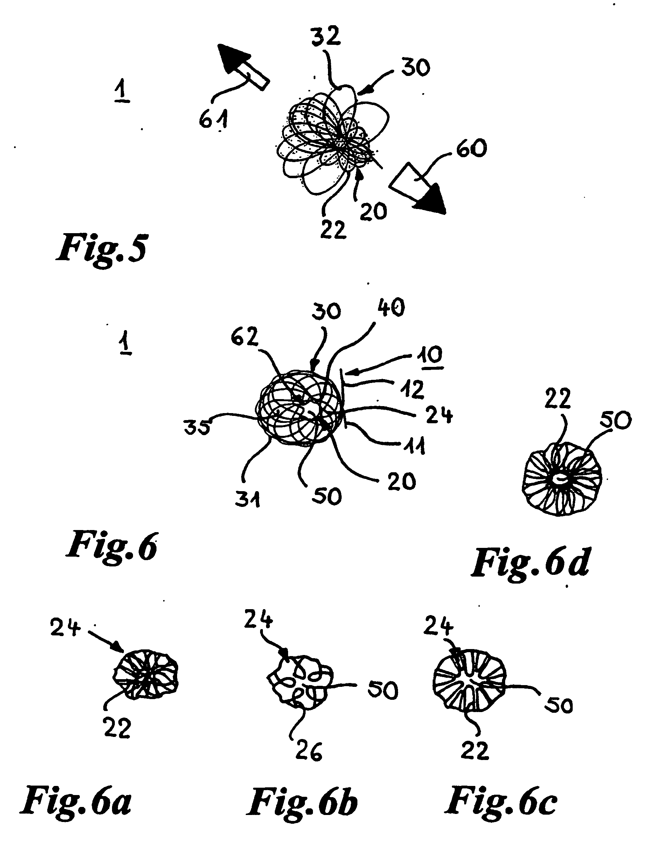

Wie insbesondere auch der Figur 3 entnommen werden kann, kann ein Rand durch Schlaufen 22. 32 gebildet werden und/oder ein als "perfekter Rand" bezeichneter Rand, bei dem die einzelnen Schlaufen sehr dicht übereinandergelegt sind, wobei in dem in Figur 3 dargestellten Fall im Randbereich 23 und 33 dadurch auch eine höhere Materialkonzentration entsteht. Hierdurch lassen sich partiell die mechanischen Eigenschaften des Randbereichs und damit auch der gesamten implantierbaren Einrichtung 1 ändern. Wie insbesondere in der Figur 6 zu sehen, kann auch ein Ringbereich um die gesamte implantierbare Einrichtung herum gebildet werden, der eine hohe Materialkonzentration und damit eine größere Stabilität aufweist als der übrige Bereich der implantierbaren Einrichtung.

In Figur 4 und 4a ist eine weitere Ausführungsform einer implantierbaren Einrichtung dargestellt. Bei dieser ist ein Teilbereich 29 des proximalen Abschnitts eingefaltet, so dass ein sehr flaches Gebilde entsteht. Auf der proximalen Seite liegt der dortige Abschnitt somit doppelt. Zwischen proximalem und distalem Abschnitt verbleibt in einem Überdeckungsbereich 41 ein spaltförmiges Teilstück, in das beispielsweise eine Wandung 3 eingeführt werden kann, um die implantierbare Einrichtung an dieser zu befestigen (siehe Figur 4a). Trotz der Tatsache, dass die implantierbare Einrichtung sehr flach ausgebildet ist, ist ein vollständiger Verschluss einer Defektöffnung oder dergleichen möglich, da der Zwischenabschnitt 40 proximal (Figur 4a) geschlossen ist. Beispielsweise eignet sich diese Ausführungsform auch zum Einbringen im Bereich einer Herzklappe, da die Einrichtung 1 im proximalen und/oder distalen Abschnitt beliebig eingefaltet werden kann, so dass eine optimale Anpassung an die einseitig geringen Platzverhältnisse im Bereich einer Herzklappe möglich ist. Wie weit der proximale und/oder distale Abschnitt jeweils eingefaltet wird, kann also von dem jeweiligen Anwendungsfall abhängig gemacht werden. Es können dabei völlig unregelmäßige Tragstrukturen entstehen, die sich dann jeweils optimal den räumlichen und Platzverhältnissen anpassen lassen.

In der in Figur 5 dargestellten Ausführungsform ist ebenfalls eine unregelmäßige Tragstruktur gebildet. Diese Draufsicht zeigt in ihrem oberen Bereich den distalen Abschnitt 30 und in ihrem unteren den proximalen 20. Der distale Abschnitt ist im Wesentlichen ausschließlich aus Schlaufen 32 gebildet. Diese Schlaufen sind jedoch nur in einem Bereich angeordnet, der etwa 250° des Umfangs abdeckt. In dem restlichen Umfangsbereich ist keine solche Schlaufe angeordnet. Mit dem proximalen Abschnitt ist dies anders. Dieser weist in Figur 5 im mit dem Pfeil 60 ganz allgemein angedeuteten Bereich Schlaufen 22 auf. Zu der Seite hin, die dem Pfeil 60 entgegengerichtet ist, in Figur 5 angedeutet mit dem Pfeil 61, sind im proximalen Abschnitt nur wenige Schlaufen 22 gebildet. Es kann somit eine besonders gute Befestigung der implantierbaren Einrichtung 1 im proximalen Bereich ermöglicht werden. Im distalen Abschnitt ist durch die in dem Bereich 60 fehlende Schlaufe oder Schlaufen eine Anpassung an ein möglicherweise herausragendes Organ oder irgendein anderes Hindernis im menschlichen oder tierischen Körper möglich, wobei dennoch ein optimaler Halt auch auf distaler Seite möglich ist, da der distale Abschnitt in Richtung des Pfeils 61 eine gute Befestigung ermöglicht.

In den Figuren 6, 6a, 6b, 6c und 6d sind weitere Ausführungsformen der implantierbaren Einrichtung dargestellt, wobei die Figuren 6a bis 6d jeweils nur Detailansichten des proximalen Abschnitts wiedergeben. Die Befestigungsseite bzw. eigentliche Befestigungsseite ist wiederum das proximale Ende 24. Dieses ist in Figur 6 sehr kurz ausgeführt und stellt im Wesentlichen nur einen etwas herausgezogenen, die Durchgangsöffnung 50 umgebenden Zwischenabschnitt 40 dar. Der distale Abschnitt ist im Randbereich mit einem "perfekten Rand" versehen. Hierbei ist herausragend das drahtartige Element 10 gezeigt, aus dem wiederum die Tragstruktur der gesamten Einrichtung 1 gebildet ist. Das aus dem Randbereich 31 herausragende Ende 11 des drahtartigen Elements 10 wird dort mit eingeflochten. Zu Anschauungszwecken ist es hier jedoch herausgezogen. Das andere Ende 12 des drahtartigen Elements 10 wird vorzugsweise ebenfalls in diesen Randbereich eingeflochten oder aber in die Fläche 35 des distalen Abschnitts.

In der in Figur 6 dargestellten Ausführungsform ist das proximale Ende 24 mit einander benachbart übergreifenden Schlaufen ausgebildet. In einem Bereich 62 sind lediglich drei Schlaufen nebeneinander angeordnet, ohne im Randbereich miteinander verschlungen zu sein oder einander zu überdecken. Der Randbereich des proximalen Abschnitt ist hier also auch unregelmäßig ausgebildet. Alternativ kann er aber auch regelmäßig ausgebildet werden, wie dies beispielsweise in Figur 6c angedeutet ist. Hierbei sind die Schlaufen 22 jeweils nebeneinander angeordnet. Alternativ können auch e-förmige Schlingen 26 gebildet werden, wie dies in Figur 6b angedeutet ist. Auch diese können nebeneinander oder einander übergreifend angeordnet werden. Eine definierte Durchgangsöffnung 50 wird durch die Ausführungsform nach Figur 6d erzeugt, bei der die Schlaufen einander übergreifen und die Öffnung 50 umgrenzen. Sowohl bei der Ausführungsform nach Figur 6b als auch bei der Ausführungsform nach Figur 6c oder 6d verbleibt weiterhin eine Durchgangsöffnung 50. Werden aber die einzelnen e-förmigen Schlingen 26 oder Schlaufen 22 völlig miteinander verschlungen, verbleibt keine Durchgangsöffnung mehr im Zentrum oder im Innern des proximalen Abschnitt bzw. dadurch auch des distalen Abschnitts (Figur 6a). In diesem Bereich kann zusätzlich noch eine Befestigungsplatte angebracht werden, um eine bessere Befestigung innerhalb einer Öffnung im menschlichen oder tierischen Körper zu gewährleisten. Die Befestigungsplatte kann sich beispielsweise auf einer Seite einer Defektöffnung anlagern. Hiermit ist dann auch ein vollständiger Verschluss eines Organweges, einer Defektöffnung oder dergleichen möglich.

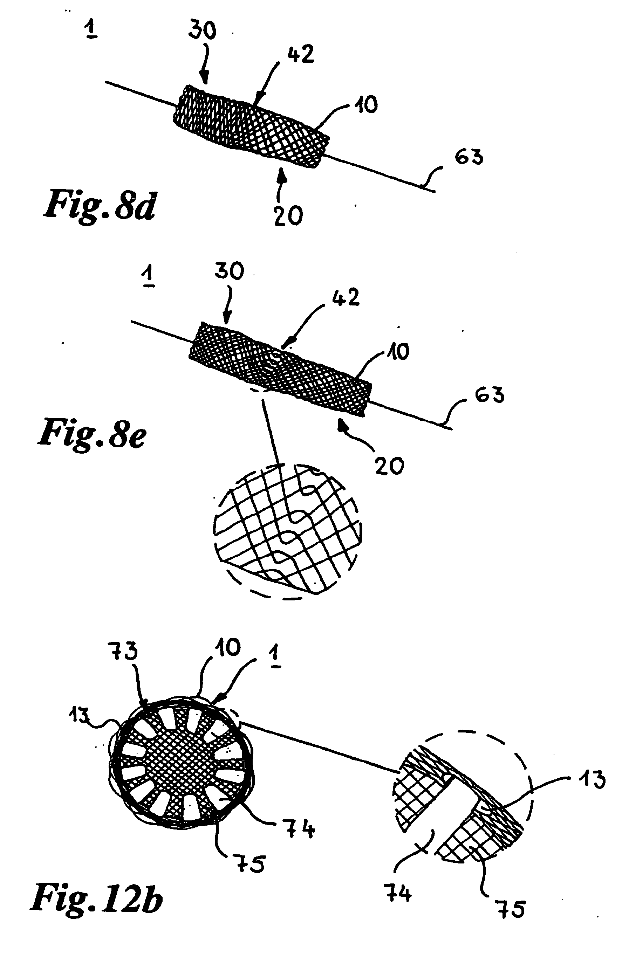

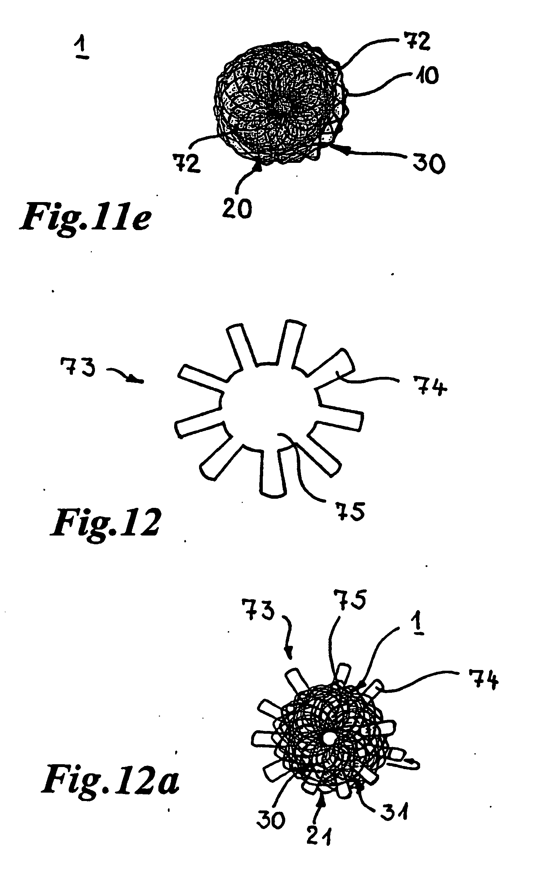

In einer weiteren alternativen Ausführungsform verbleibt nur der Randbereich des distalen Abschnitts und des proximalen Abschnitts. Ein solcher Ring ist als Tragstruktur für eine Membran in Figur 12b zu sehen. Der proximale Abschnitt ist so weit nach außen gezogen, dass er im Durchmesserbereich des distalen Abschnitts liegt. Hierbei entsteht ein sehr stabiler Ring, der mit einer Innenmembran versehen werden kann oder auch als Versteifungsmittel ohne eine solche Membran in einen Organweg oder dergleichen im menschlichen oder tierischen Körper eingesetzt werden kann.

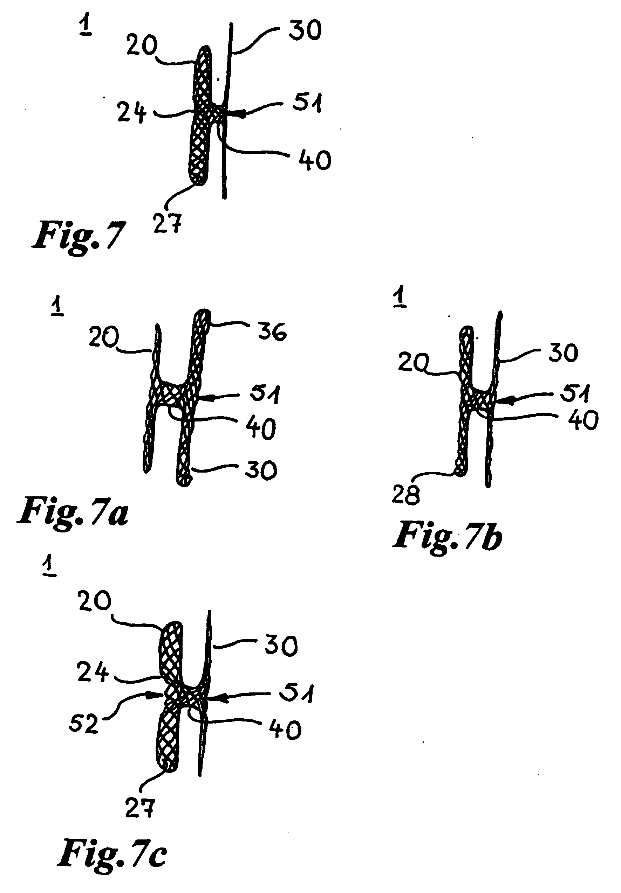

In den Figuren 7. 7a, 7b und 7c sind weitere Ausführungsvarianten des proximalen Abschnitts 20 dargestellt. Der distale Abschnitt ist dabei jeweils im Wesentlichen scheibenförmig ausgebildet. Er kann jedoch ebenfalls in einer alternativen Ausführungsform entsprechend den proximalen Abschnitten ausgebildet werden. Figur 7 zeigt dabei eine Seitenansicht einer Ausfürungsform gemäß der Erfindung, mit eine proximalen Abschnitt, der zunächst scheibenförmig nach außen gezogen und nachfolgend wieder nach innen umgebogen ist und am proximalen Ende 24 im Bereich der Durchgangsöffnung miteinander verschlungene Enden von Schlaufen und Schlingen aufweist. Eine Durchgangsöffnung 51 ist dabei nur im Bereich des distalen Abschnitts 30 und des Zwischenabschnitts 40 gebildet. Sie endet innerhalb eines durch den proximalen Abschnitt 20 gebildeten Innenraums 27.

Die Ausführungsform gemäß Figur 7c unterscheidet sich von der gemäß Figur 7 dadurch, dass das proximale Ende 24 des proximalen Abschnitts 20 nicht vollständig verschlossen ist, d.h. die dort endenden Schlaufen und Schlingen nicht einander überdecken, sondern zwischen sich eine Öffnung 52 belassen. Weiterhin wird von dem proximalen Abschnitt ein Innenraum 27 umgrenzt. Die im distalen Abschnitt 30 beginnende Durchgangsöffnung 51 setzt sich durch den Zwischenabschnitt 40 in die Öffnung 52 im proximalen Abschnitt fort. Sofern die Öffnungen nicht noch durch eine Membran verschlossen werden, ist nun ein Teilverschluss nach Implantation dieser Einrichtung 1 möglich.

Ein noch besserer Durchtritt von Flüssigkeiten ist durch die in Figur 7b dargestellte Einrichtung 1 möglich. Bei dieser ist der Rand des proximalen Abschnitts, d.h. das proximale Ende, nicht bis zu der Durchgangsöffnung hin zusammengezogen. Vielmehr ist der proximale Abschnitt lediglich dadurch gebildet, dass zuerst dieser von dem Zwischenabschnitt 40 tellerartig nach außen geformt und dann am Rand wieder ein Stückchen nach innen gezogen ist. Dieser Randbereich 28 ähnelt einer Krempe. Es wird kein Innenraum mehr umgrenzt bzw. nur noch in diesem Randbereich. Eine diesbezüglich noch extremere Form ist in Figur 7a gezeigt. Hierbei ist der proximale Abschnitt im Wesentlichen wiederum nur tellerförmig und lediglich ganz leicht proximal nach außen gebogen. Der distale Abschnitt ist ebenfalls ein wenig distal nach außen gebogen. Dies kann auch über den Umfang des distalen Abschnitts unterschiedlich erfolgen, wie in Figur 7a in dem Randbereich 36 angedeutet. Hier ist lediglich auf einem Teilstück des Randes dieser nach innen hineingefaltet bzw. umgekrempelt, so dass eine Krempe partiell am Umfang des distalen Abschnitts gebildet wird.

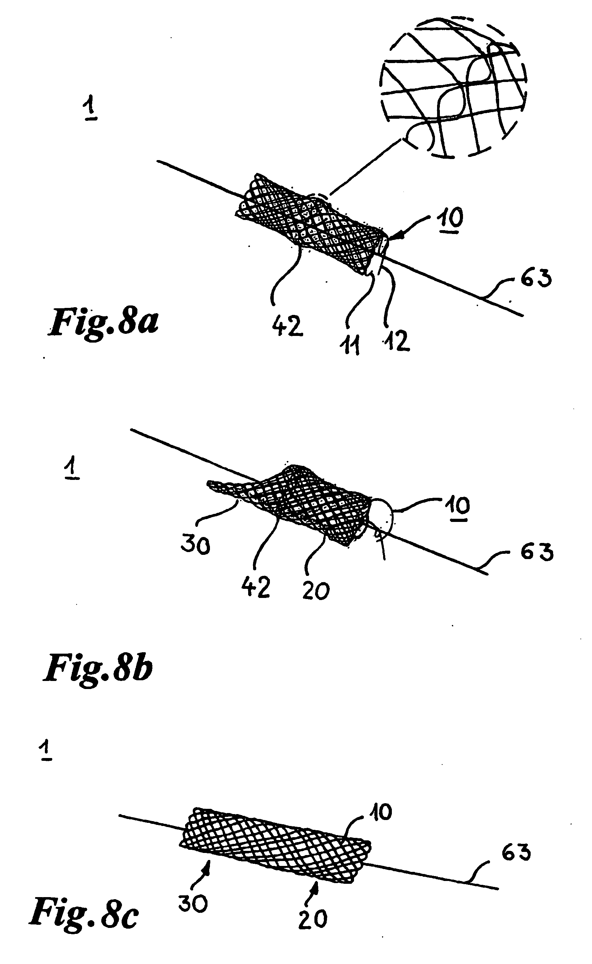

Die Figuren 8a bis 8e zeigen jeweils Varianten von Grundwickelformen der Tragstruktur einer erfindungsgemäßen implantierbaren Einrichtung 1. Die jeweiligen Grundwickelformen sind im Wesentlichen schlauchförmig und bei Verwendung nur eines drahtartigen Elementes 10 aus zwei aneinandergesetzten Hälften aufgebaut. In einem Anschlussbereich 42 sind die beiden Hälften aneinandergefügt, jeweils weiterhin unter Verwendung nur des einen drahtartigen Elementes 10. In diesem Anschlussbereich 42 wird dadurch eine größere Steifigkeit der Grundwickelform erzielt. Dies kann sich bei bestimmten Anwendungsfällen als besonders vorteilhaft erweisen, da auch nach dem Umformen in die gewünschte Sekundärform in diesem Bereich ein guter Halt möglich ist. Beispielsweise bei einfachem Zusammenschieben in Richtung der Längsachse 63 kann bei Figur 8a eine ringförmige Tragstruktur entstehen, die im Randbereich einander übergreifende Schlaufen aufweist, sowohl auf der proximalen als auch auf der distalen Seite. Im Bereich zwischen der proximalen und der distalen Seite bzw. deren Enden, also im Anschlussbereich 42, ragt ebenfalls ein Kranz von herausstehenden Schlaufen aus dem Ring heraus. Diese später herausragenden Schlaufen der verketteten Enden der aneinandergefügten Hälften sind in dem Detailausschnitt in Figur 8a besonders gut zu sehen. Außerdem sind zahlreiche andere Formgebungen als sekundäre Formen möglich, wie beispielsweise in den vorstehenden und nachfolgenden Figuren noch gezeigt. Nach Figur 8a sind die beiden Enden 11, 12 des drahtartigen Elementes auf einer Seite aus der schlauchförmigen Grundwickelform herausgeführt.

Figur 8b stellt eine Variante zur Figur 8a dar, wobei die zweite Hälfte dieser Einrichtung nur halbseitig ausgebildet ist, d.h. das drahtartige Element 10 ist dort so gewickelt, dass der in diesem Falle distale Abschnitt 30 ab dem Anschlussbereich 42 nur noch partiell ausgebildet ist. Umgekehrt kann der partiell gewickelte Bereich auch der proximale Abschnitt sein. Auch können beliebige andere Formgebungen hier erzielt werden. Beispielsweise können beide Abschnitte 20, 30 lediglich partiell ausgebildet sein. Die Formgebung hängt hier besonders von der jeweiligen Anwendungsform ab bzw. welche Sekundärform erzielt werden soll. Im Anschlussbereich 42 kann jeweils, sofern dies gewünscht wird, durch entsprechendes Wickeln eine Versteifung vorgesehen werden, so dass ein guter Halt an der Implantationsstelle ermöglicht wird.

Die Figur 8c zeigt eine symmetrische Ausbildung der Grundwickelform. d.h., beide Hälften, also proximaler und distaler Abschnitt 20, 30 sind symmetrisch zueinander gewickelt. Vorzugsweise erfolgt der Wickelvorgang in einem Zug, ohne Ausbilden zweier Hälften. Eine weitere Variante ist in Figur 8d gezeigt, wobei bei dieser zwei aneinandergearbeitete Hälften aus einem drahtartigen Element als Grundwickelform vorgesehen sind. Die beiden Hälften, also der proximale und der distale Abschnitt 20. 30, weisen jeweils verschiedene Winkel hinsichtlich der Wickel richtung auf. Der Winkel im distalen Abschnitt 30 beträgt hier etwa 180°, wohingegen der Wickelwinkel im proximalen Abschnitt etwa 90° beträgt. Selbstverständlich sind beliebige andere Winkelwerte möglich. Im Anschlussbereich sind diese beiden Hälften zu einem Stück aneinandergesetzt, wobei für die gesamte Grundwickelform lediglich ein drahtartiges Element 10 verwendet ist.

Eine weitere Variante ist in Figur 8e dargestellt. Hierbei sind die beiden Hälften der Grundwickelform, die den proximalen und distalen Abschnitt 20, 30 bilden, so aneinandergesetzt, dass jeweils beim Wickelvorgang eine Umkehrung der Wickelrichtung im Anschlussbereich 42 vorgesehen ist. Dies kann besser dem Detailausschnitt in Figur 8e entnommen werden. Auch hierdurch können, wie bereits bei der Ausführungsform nach Figur 8d, insbesondere hinsichtlich der Steifigkeit der Sekundärform der Tragstruktur, besondere Effekte erzielt werden, insbesondere lediglich partielle Versteifungen in der Tragstruktur vorgesehen werden.

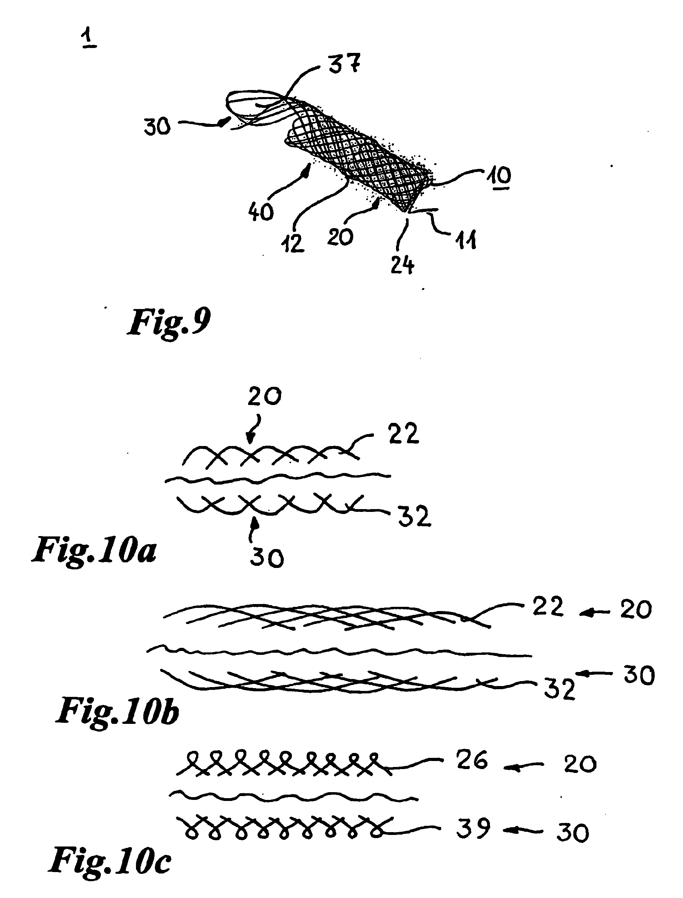

Figur 9 gibt eine weitere Variante einer solchen aus mehreren Teilen zusammengesetzten Tragstruktur einer Einrichtung 1 wieder die nicht zur Erfindung gehört. Hierbei ist der erste Teil, hier beispielhaft der proximale Abschnitt 20, zylindrisch geformt, der Zwischenabschnitt 40 ist zwar im Wesentlichen zylindrisch geformt, weist jedoch einige Lücken auf. Der distale Abschnitt 30 besteht lediglich aus vier miteinander verbundenen Schlaufen, so dass dort große Öffnungen 37 verbleiben. In diese kann beispielsweise, sofern die Einrichtung zum Einfangen von Gegenständen innerhalb des menschlichen Körpers, wie beispielsweise Steinen etc., verwendet wird, zum Ergreifen und Festhalten dieser Gegenstände verwendet werden. Diese Ausführungsform gemäß Figur 9 zeigt, dass beliebige Einzelabschnitte aneinandergesetzt werden können, die alle zusammen aus lediglich einem drahtartigen Element geformt sind. Bevorzugt werden dann die beiden Enden 11, 12 des drahtartigen Elementes lediglich auf einer Seite, insbesondere am proximalen Ende des proximalen Abschnitts herausgeführt. Hierdurch kann die Verletzungsgefahr für den Patienten bestmöglich eingeschränkt werden. Alternativ können die Enden jedoch auch in der Mantelfläche beispielsweise des proximalen Abschnitts oder der anderen Abschnitte verflochten werden.

Figur 10 zeigt in seinen Teilen a, b und c jeweils Ausbildungsmöglichkeiten für Ränder von distalem und proximalem Abschnitt. Hierbei können die Schlaufen 22, 32 jeweils mit ihren benachbarten Schlaufen verschränkt (Figur 10a), langgezogen mit den benachbarten Schlaufen überkreuzt (Figur 10b) oder e-förmig ausgebildet sein (Figur 10c). Auch zahlreiche Zwischenformen sind möglich. Hierbei kann die Wahl der Randform abhängig gemacht werden von dem jeweiligen Anwendungsfall.

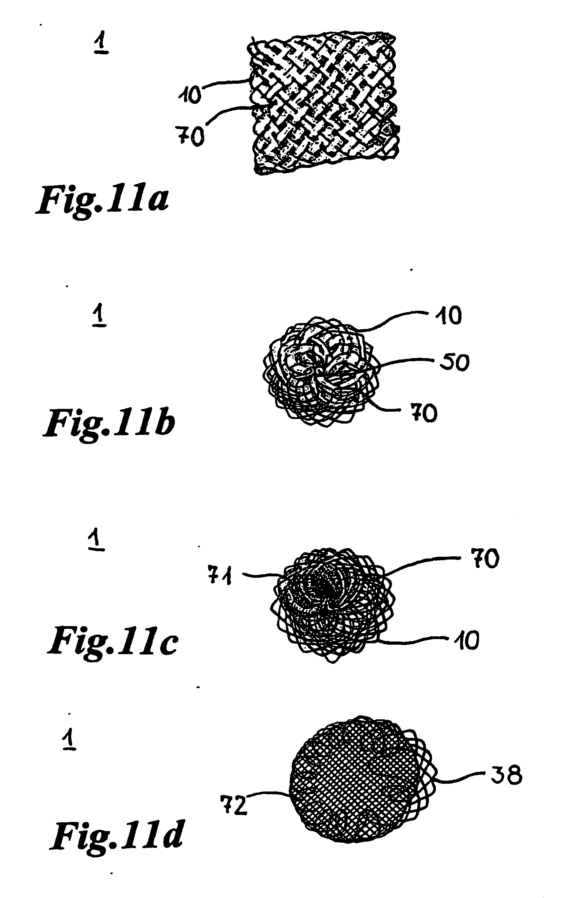

In Figur 11a bis 11e sind verschiedene Ausführungsvarianten von erfindungsgemäßen implantierbaren Einrichtungen 1 dargestellt, die Membranen, membranbildende oder membranartige Strukturen aufweisen. Figur 11a zeigt eine seitliche Draufsicht auf eine schlauchartige Grundwickelform einer aus lediglich einem drahtartigen Element hergestellten implantierbaren Einrichtung 1, bei der ein weiterer Faden 70 in die Tragstruktur der Einrichtung 1 eingewoben ist. Dadurch, dass die Materialstärke des drahtartigen Elementes 10 und des Fadens 70 sich sehr stark unterscheiden, der Faden 70 viel dicker ist als das drahtartige Element, kann eine besonders dichte membranartige Struktur gebildet werden. Wird eine solche Primärform, wie sie in Figur 11a dargestellt ist, in eine Sekundärform umgewandelt, kann beispielsweise die in Figur 11b gezeigte Variante entstehen. Diese ähnelt im Wesentlichen der in der Figur 1 dargestellten. Im Unterschied zu dieser ist jedoch die in Figur 11b dargestellte Variante im Wesentlichen flüssigkeitsundurchlässig, insbesondere auch im Bereich der Durchgangsöffnung 50, da der Faden 70 durch die Verflechtung mit dem drahtartigen Element eine Membran bildet. Je nach Materialwahl für den Faden 70 kann hierdurch beispielsweise eine Defektöffnung im Herzen eines Menschen verschlossen werden und dort ggf. sogar Gewebe aufwachsen.

Eine Alternative zu dem vollständigen Verschließen der Tragstruktur, die durch das drahtartige Element 10 gebildet wird, durch den Faden 70, bei der lediglich ein Teilbereich mit diesem durchflochten ist, ist in Figur 11c dargestellt. Hierbei ist insbesondere die Durchgangsöffnung 50 verschlossen, so dass ggf. auch ein vollständiger Verschluss einer im Vergleich zu den Abmessungen der implantierbaren Einrichtung kleineren Defektöffnung möglich ist. Andererseits kann diese Ausführungsvariante jedoch auch lediglich zum Teilverschluss von Defektöffnungen verwendet werden, da der verflochtene Faden lediglich im Bereich 71 angeordnet ist.

In einer weiteren alternativen Ausführungsform, wie sie in Figur 11d dargestellt ist, ist kein Faden mehr in die Tragstruktur eingeflochten, sondern in diese ein Gewebe oder Gelege als Membran 72 eingefügt. In der in Figur 11d dargestellten Ausführungsvariante füllt diese Membran 72 jedoch nicht die gesamte Fläche von distalem und proximalem Abschnitt der Einrichtung 1 aus, sondern belässt einen Randbereich 38 (in dem dargestellten Fall), in dem keine Membran angeordnet ist. Letztere ist in dem dargestellten Fall lediglich in den proximalen Abschnitt integriert, kann in einer alternativen Ausführungsform jedoch ebenso in den distalen Abschnitt integriert werden, wobei dann auch im proximalen Abschnitt ein Randbereich verbleiben kann, den die Membran nicht überdeckt.

Bei einer weiteren alternativen Ausführungsform, wie sie in Figur 11e dargestellt ist, sind sowohl der proximale als auch der distale scheibenförmige Abschnitt mit einer solchen Membran 72 versehen. Die beiden proximalen und distalen Abschnitte sind zueinander versetzt angeordnet, also der Zwischenabschnitt mit der Durchgangsöffnung nicht zentral in den beiden proximalen und distalen Abschnitten angeordnet, so dass auch die beiden Membranen sich in der Draufsicht lediglich teilweise überdecken. Dies soll zeigen, dass es möglich ist, Membranen auf der Tragstruktur der implantierbaren Einrichtungen beliebig aufzubringen.

In den Figuren 12, 12a und 12b sind weitere Ausführungsformen einer erfindungsgemäß ausgeführten Membran 73 dargestellt. Diese weist um ihren Umfang verteilt herauskragende Arme 74 auf. Eine Befestigung an dem proximalen und/oder distalen Abschnitt einer implantierbaren Einrichtung erfolgt dadurch, dass die Arme entweder durch die durch das drahtartige Element 10 gebildeten Maschen und/oder durch Schlaufen oder Schlingen im Randbereich des distalen und/oder proximalen Endes gefädelt werden. Die Arme werden dann vorzugsweise nach dem Hindurchstecken aufeinander gelegt und auf der inneren Membranfläche 75 befestigt, insbesondere durch Vernähen, Kleben, Verschweißen, Crimpen oder eine andere Befestigungsart. Grundsätzlich kann auch jede andere Art von Membran im Randbereich des proximalen und/oder distalen Abschnitts auf diese Art und Weise durch eine mechanische Befestigung befestigt werden. Die Membran oder membranbildende Struktur oder membranartige Struktur kann in jedem Bereich innerhalb der Tragstruktur der implantierbaren Einrichtung 1 positioniert werden, insbesondere im proximalen Abschnitt und/oder distalen Abschnitt und/oder im Zwischenabschnittsbereich.

Figur 12a zeigt die Membran 73 nach Figur 12 in an einer Tragstruktur einer implantierbaren Einrichtung 1 befestigter Position. Hierbei ist die Tragstruktur auf die Membran aufgelegt und die herausragenden Arme 74 um den Rand 21 der Tragstruktur der implantierbaren Einrichtung herumgelegt und auf der Innenseite, die zu dem distalen Abschnitt 30 weist, befestigt. Im dargestellten Fall sind die Arme 74 an ihren jeweiligen Enden durch die Tragstruktur im proximalen Abschnitt 20 hindurch an der auf der proximalen Seite der Tragstruktur angeordneten Membranfläche 75 angenäht. In der dargestellten Ausführungsform gemäß Figur 12a ist somit die Membran 73 lediglich an dem proximalen Abschnitt befestigt. Es kann ebenso auch eine Befestigung an dem distalen Abschnitt oder an beiden erfolgen. Vorzugsweise wird die Membran so befestigt, dass ein problemloses Absetzen und Arretieren der implantierbaren Einrichtung weiterhin gewährleistet wird.

In Figur 12b ist eine weitere Ausführungsform einer Tragstruktur mit eingefügter Membran 73 gezeigt. Die Tragstruktur ist dabei ringförmig und kann insbesondere aus einem geschnittenen Rohr gebildet sein, jedoch ebenfalls aus einem gewickelten drahtartigen Element 10. Die Arme 74 sind durch Ösen 13 hindurch gesteckt und, wie bereits in Figur 12a, auf der Mantelfläche 75 befestigt, insbesondere durch Vernähen, Verkleben etc. Die Ösen 13 können entweder in einem geschnittenen Rohr gebildet sein, das die Tragstruktur bildet, oder beim Wickeln durch das drahtartige Element. Innerhalb der Tragstruktur sind beliebig viele solcher Ösen gebildet, wobei zur Befestigung selbstverständlich grundsätzlich auch die Schlaufen am Rand der Tragstruktur verwendet werden können, um durch diese die Arme 74 der Membran hindurch zu stecken und somit dort die Membran zu befestigen.

Eine weitere Variante, die in den Figuren nicht dargestellt ist, ist das Tauchen der Tragstruktur, um eine membranartige Struktur auszubilden. Hierbei wird die Tragstruktur insbesondere in ein filmbildendes Material getaucht, wonach in der Tragstruktur ein Film verbleibt, der membranartig ist. Je nach Materialwahl des Tauchmediums können so bestimmte Effekte der Membran erzielt werden, insbesondere eine hydrophobe oder hydrophile Oberfläche, besonders um das Aufwachsen von Gewebe zu erleichtern. Auch eine Luft- und/oder Wasserdurchlässigkeit kann durch diese Materialwahl erzielt werden. Insbesondere eignen sich für das Tauchmaterial ein oder mehrere Monomere, die ein natürliches oder synthetisches Polymer bilden, insbesondere durch Polyaddition. Polymerisation oder Polykondensation. Besonders geeignen sind dabei Polycarbonate, Polyester, Polyamide. Polyolefine oder auch Polyurethan. Auch natürliche Harze eignen sich, soweit sie filmbildend sind.

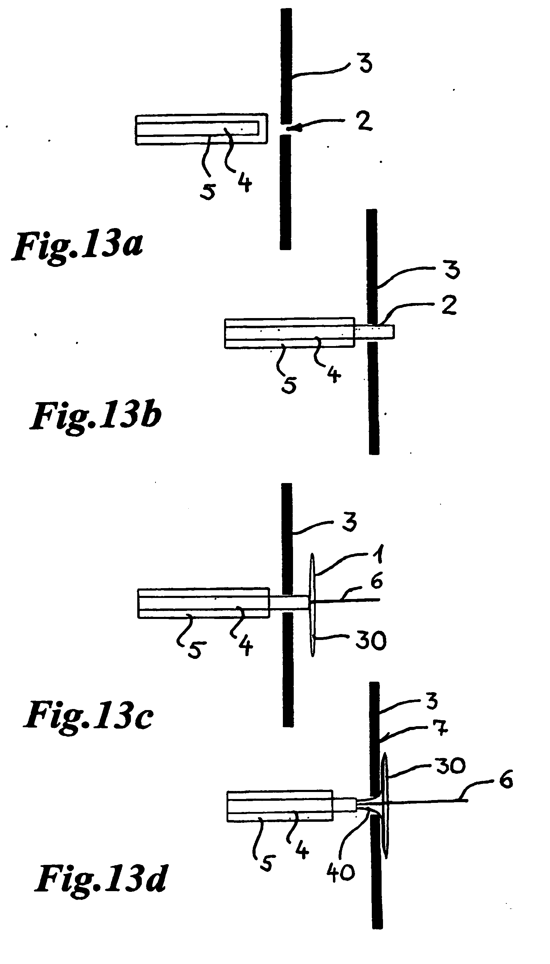

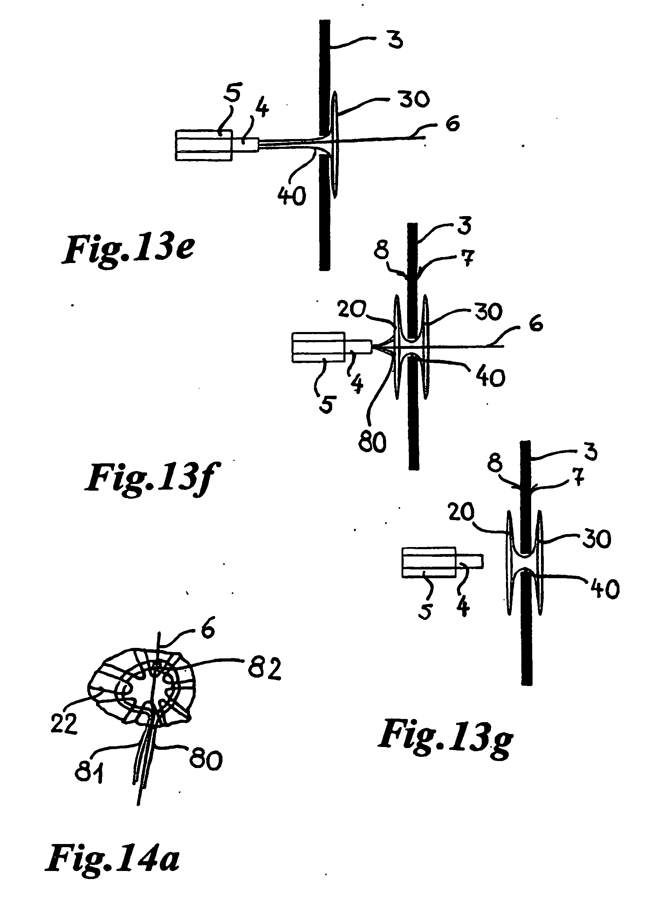

In den Figuren 13a bis 13g ist der Abwurf der implantierbaren Einrichtung 1 dargestellt. Hierbei wird die implantierbare Einrichtung in der Defektöffnung 2 in der Wandung 3 angebracht. Zu diesem Zweck wird ein Katheter 5 mit, einem Vorschubröhrchen 4 darin in den Bereich der Defektöffnung 2 gebracht. Im zweiten Schritt (Figur 13b) wird das Vorschubröhrchen 4 durch die Öffnung 2 hindurchgeführt. Im dritten Schritt wird die implantierbare Einrichtung 1 aus dem Vorschubröhrchen 4 herausgeschoben, wobei diese auf einem Führungsdraht 6 befestigt ist. Es entfaltet sich zunächst der distale Abschnitt 30 der implantierbaren Einrichtung. Im weiteren Schritt (Figur 13d) wird das Vorschubröhrchen 4 aus der Öffnung 2 zurückgezogen und der Zwischenabschnitt 40 entfaltet. Dabei lagert sich der distale Abschnitt 30 auf der distalen Außenseite 7 der Wandung 3 an. Im weiteren Zurückziehen von Vorschubröhrchen 4 und Katheter 5 wird die implantierbare Einrichtung 1 weiter auf dem Führungsdraht aus dem Vorschubröhrchen 4 herausgeschoben, zunächst noch in langgestreckter Form. An dem proximalen Abschnitt befestigte Haltedrähte 80, wie sie besser in Figur 14a bis 14c zu sehen sind, sind noch an dem Führungsdraht und in dem Vorschubröhrchen angeordnet. In dem nächsten und vorletzten Aussetzschritt, der in Figur 13g gezeigt ist, ist die implantierbare Einrichtung vollständig aus dem Vorschubröhrchen herausgeschoben. Die Haltedrähte sind noch mit dem proximalen Abschnitt 20 verbunden. Im letzten Schritt, der in Figur 13f gezeigt ist, sind der Führungsdraht und die Haltedrähte ebenfalls in das Vorschubröhrchen zurückgezogen. Die implantierbare Einrichtung hat vollständig ihre Sekundärform angenommen, bei der der proximale Abschnitt 20 auf der proximalen Außenseite 8 und der distale Abschnitt 30 auf der distalen Außenseite 7 der Wandung 3 liegt.

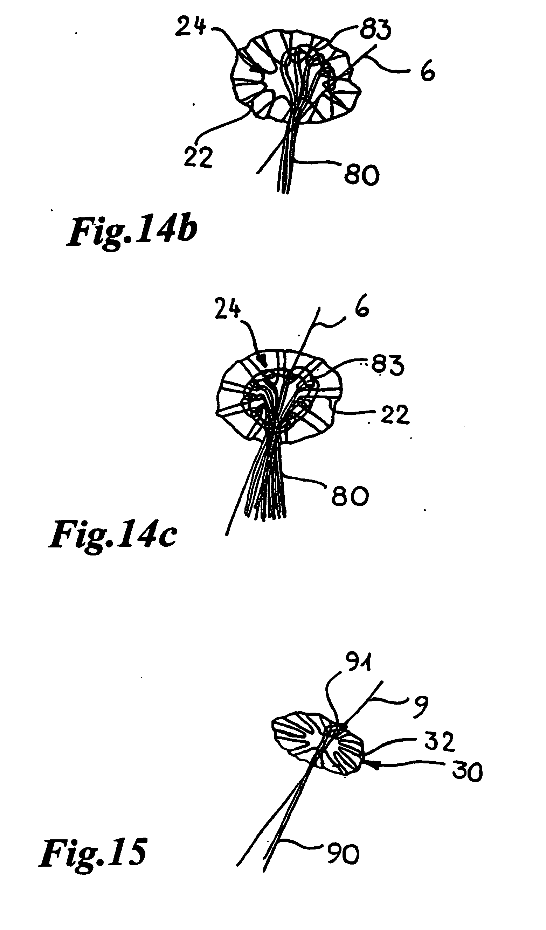

Die Figuren 14a bis 14c zeigen die Anordnung von Haltedrähten, die zu dem Platziersystem, wie es in den Figuren 13a bis 13g gezeigt ist, gehören. Es kann lediglich ein Haltedraht durch alle Schlaufen oder Schlingen am proximalen Ende und/oder distalen Ende gefädelt sein unter Bilden einer Schlaufe zum Durchführen des Führungsdrahtes oder ohne Schlaufenbildung. Die Ausführungsform mit nur einem Draht, der durch alle diese Schlingen gefädelt ist, ist in den Figuren 14 nicht dargestellt. Dargestellt ist jedoch in Figur 14a die Ausführungsvariante, bei der zwei Haltedrähte 80, 81 jeweils durch die Hälfte der vorhandenen Schlingen bzw. Schlaufen gezogen sind, wobei der Führungsdraht 6 durch eine von beiden Haltedrähten 80, 81 gebildete Schlaufenöffnung 82 gefädelt ist. Die beiden Haltedrähte sind in diesem Bereich zur Schlaufe ausgebildet doppelt gelegt. Beide Schlaufen überdecken in einem bestimmten Bereich einander und bilden zwischen sich die Schlaufenöffnung 82. Dadurch, dass der Führungsdraht durch diese Schlaufenöffnung 82 hindurchgeführt ist und an den beiden Haltedrähten gezogen wird, wird eine feste Einheit zwischen den beiden Haltedrähten, dem Führungsdraht und der implantierbaren Einrichtung erzielt.

In einer alternativen Ausführungsform, wie sie in Figur 14b dargestellt ist, wird eine Kette aus Haltedrähten gebildet, wobei diese jeweils ineinandergreifende Schlaufen 83 aufweist. Der Führungsdraht wird in der in Figur 14b dargestellten Ausführungsform lediglich durch die letzte Schlaufe 83 gefädelt und hält damit auch alle anderen fest, da diese aneinanderhängen. In der in Figur 14b dargestellten Variante sind lediglich vier Haltedrähte vorgesehen, wohingegen in der in Figur 14c dargestellten Variante durch jede der Schlaufen 22 am proximalen Ende der Einrichtung jeweils ein Haltedraht 80 gefädelt ist. Diese sind alle miteinander verkettet, wobei durch die letzte Schlaufe 83 wiederum der Führungsdraht geschoben ist. Durch Zug an den Haltedrähten entsteht wiederum zwischen diesen, dem Führungsdraht 6 und den Schlaufen am proximalen Ende 24 der implantierbaren Einrichtung eine Einheit, so dass ein Dirigieren der implantierbaren Einrichtung durch den Führungsdraht und entlang diesem möglich ist. Sind alle Schlingen/Schlaufen am proximalen Ende (oder auch am distalen Ende) in einem Punkt zusammengeführt (im Bereich der Durchgangsöffnung), reicht im Zweifel das Vorsehen lediglich eines Haltedrahtes und damit einer Schlaufe und eines Führungsdrahtes aus, um eine feste Verbindung zum Dirigieren der implantierbaren Einrichtung zu schaffen. Ist hingegen eine durchaus merkliche Durchgangsöffnung zwischen den Schlaufen bzw. Schlingen am proximalen/distalen Ende der Einrichtung 1 vorgesehen, werden vorteilhaft mehr als ein Haltedraht verwendet. Beispielsweise können beim Vorsehen von 24 Schlingen bzw. Schlaufen am proximalen Ende der Einrichtung auch 24 Haltedrähte vorgesehen werden. Grundsätzlich können sogar noch mehr Haltedrähte vorgesehen werden, sofern dies vorteilhaft erscheint.

Sofern es sich herausstellt, dass die implantierbare Einrichtung wieder von dem Implantationsort entfernt werden soll, insbesondere, da der zu erzielende Effekt mittlerweile eingetreten ist, oder da die implantierbare Einrichtung an der falschen Stelle implantiert wurde, ist es erfindungsgemäß auch möglich, diese mit dem Platziersystem, wie es vorstehend ausgeführt ist, wieder zu entfernen und/oder zu extrahieren. Zu diesem Zweck sind ein Führungsdraht 9 und zumindest ein Extraktionsdraht 90 vorgesehen. Der Führungsdraht 9 kann jedoch auch der in den vorstehenden Figuren dargestellte Führungsdraht 6 sein. Auch der Extraktionsdraht 90 kann identisch sein mit einem Haltedraht 80. Für die Extraktion der implantierbaren Einrichtung wird der Extraktionsdraht durch deren Tragstruktur hindurch auf die distale Seite geschoben, wobei der Extraktionsdraht zur Schlaufe gelegt ist. Der Führungsdraht 9 wird ebenfalls durch die Tragstruktur hindurch auf die distale Seite der implantierbaren Einrichtung 1 geschoben und durch die Schlaufe 91 des Extraktionsdrahtes gefädelt. Nachfolgend wird zunächst an dem Extraktionsdraht 90 und anschließend an dem Führungsdraht zusammen mit dem Extraktionsdraht gezogen. Hierdurch halten sich der Extraktionsdraht an dem Führungsdraht und beide zusammen an der Tragstruktur der implantierbaren Einrichtung 1 fest und können diese in einen Katheter hineinziehen.

Um nicht nur ein Platzieren, sondern auch ein Extrahieren der implantierbaren Einrichtung zu ermöglichen, wird vorzugsweise ein Verkaufsset mit einem solchen Platziersystem mit Haltedrähten, Führungsdraht. Vorschubröhrchen und Katheter sowie ggf. verschiedenen Ausführungsformen der implantierbaren Einrichtungen zusammen angeboten.

Anstelle des Vorsehens des drahtartigen Elementes kann die gesamte implantierbare Einrichtung 1 auch aus einem geschnittenen Rohr gebildet werden, dies gehört nicht zur Erfindung. Dieses bzw, das drahtartige Element gemäß Figur 1 bis 14c kann außerdem chemisch und/oder mechanisch behandelt, insbesondere geätzt, elektropoliert, mikrogeschliffen oder anderweitig behandelt sein. Dies kann auch zumindest in Teilbereichen erfolgen. Hierdurch können ebenfalls membranartige Strukturen gebildet werden. Unabhängig davon, ob ein drahtartiges Element oder ein (laser-)geschnittenes Röhrchen verwendet werden, bestehen diese bevorzugt aus einem biokompatiblen Material, insbesondere einem Metall oder einer Metalllegierung, insbesondere Edelstahl, oder einem Kunststoff, wie Polycarbonat, insbesondere einem Formgedächtnismaterial, wie Nitinol. Die Membranen, membranbildenden oder membranartigen Strukturen können ebenfalls aus einem natürlichen oder synthetischen Stoff bestehen, insbesondere einer Gaze aus einem Polymer oder aus Baumwolle oder einem anderen natürlichen Material. Dacronfäden und Carbonfasern eignen sich ebenfalls. Auch hierbei wird vorzugsweise auf die Biokompatibilität des Materials geachtet. Insbesondere relevant ist die erfindungsgemäße implantierbare Einrichtung im Bereich von VSD und ASD, also arteriellem Septumdefekt und Ventrikularseptumdefekt.

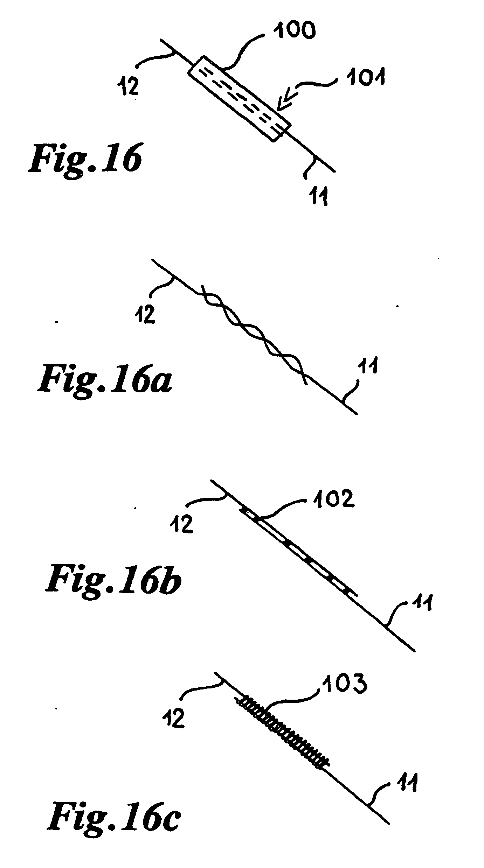

Die Figuren 16 bis 16c zeigen verschiedene Varianten, um die beiden Drahtenden 11, 12 des drahtartigen Elementes 10 miteinander zu verbinden, um ein Verletzen des die implantierbare Einrichtung umgebenden Gewebes des menschlichen oder tierischen Körpers zu vermeiden. Wie in Figur 16 dargestellt, ist zum einen das Verbinden dieser beiden Enden 11, 12 durch eine Hülse 100 möglich. Z.B. an der mit den beiden Pfeilen gekennzeichneten Stelle 101 kann eine Kraft aufgebracht werden, um die Hülse dort zu verpressen. Dies kann beispielsweise mit einem Hammer, einer Presszange oder dergleichen Hilfsmittel erfolgen. Auch ein Verkleben innerhalb der Hülse ist möglich, wobei beispielsweise nach Einführen der beiden Enden 11, 12 in die Hülse diese mit einem Klebstoff verfüllt wird. Auch ein Verschweißen oder Verlöten der Enden der Hülse ist möglich oder ein beliebiges anderes Verschließen von dieser.

Eine andere Variante des Zusammenfügens der Drahtenden 11, 12 ist in Figur 16a dargestellt. Hierbei sind die beiden Enden miteinander vertwistet, so dass ein Halt gegen ungewolltes Öffnen gegeben ist. Üblicherweise reicht bereits ein solches Vertwisten der Drahtenden gegeneinander aus, um ein ungewolltes Lösen zu verhindern. Zusätzlich kann ggf. jedoch noch zumindest ein Schweiß- oder Lötpunkt gesetzt werden, der die beiden Drahtenden noch fester miteinander verbindet.

Auch ein Laserschweißen oder Verkleben der Drahtenden miteinander, wie es in Figur 16b dargestellt ist, ist möglich. Hierbei sind mehrere Verbindungspunkte 102 durch Punktschweißen, Kleben oder dergleichen zwischen den beiden Drahtenden angebracht.

Eine weitere alternative Lösung des Verbindens der beiden Drahtenden ist in Figur 16c dargestellt. Hierbei werden die Enden 11, 12 des drahtartigen Elementes miteinander über eine sehr feine Spirale 103 verbunden. Diese kann auch als Mikrospirale bezeichnet werden. Zusätzlich wird vorzugsweise eine Klebeverbindung zwischen der Spirale und den Enden 11, 12 vorgesehen, um ein ungewolltes Lösen der Verbindung wirkungsvoll zu verhindern. Selbstverständlich sind noch beliebige andere Varianten des Verbindens der Enden des drahtartigen Elementes möglich, insbesondere auch Kombinationen der in den Figuren 16 bis 16c dargestellten Ausführungsformen. Auch das bereits vorstehend erwähnte Einflechten in die Tragstruktur ist grundsätzlich möglich. Auch hierbei können die Drahtenden jeweils noch in der Tragstruktur durch Kleben, Schweißen, Verpressen etc. zusätzlich fixiert werden.

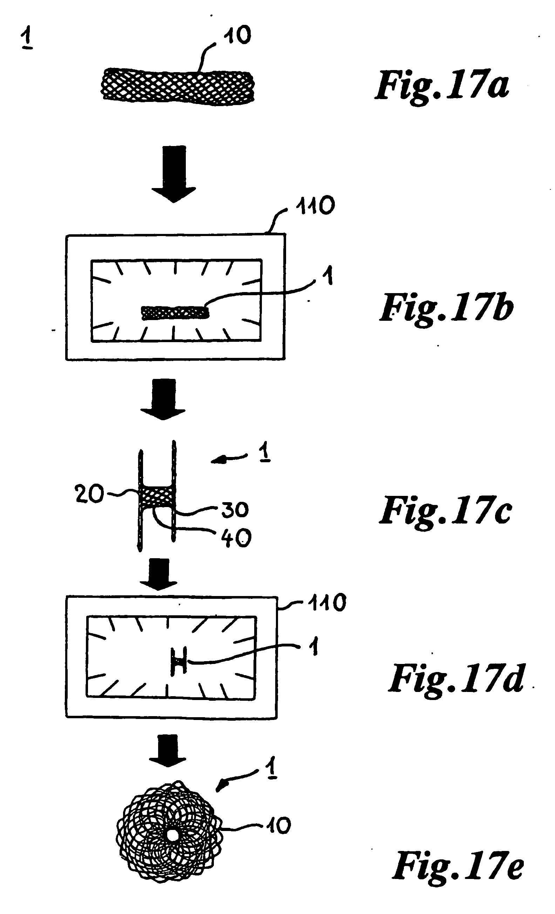

In den Figuren 17a bis 17e ist der prinzipielle Ablauf der Herstellung einer implantierbaren Einrichtung gezeigt. Hierbei wird zunächst, wie in Figur 17a angedeutet, eine Grundwickelform der Tragstruktur gefertigt. Die Fertigung erfolgt vorzugsweise per Hand durch verschränkendes Wickeln eines drahtartigen Elementes bzw. Schneiden eines rohrförmigen Elementes in entsprechender Weise, um ein Gewebe, Gelege, Netz oder eine entsprechende Tragstruktur zu erhalten. In einem zweiten Schritt wird die Grundwickelform der Tragstruktur in einem Ofen 110 geglüht, um die Grundwickelform zu stabilisieren. Als Material für die Tragstruktur wird ein Formgedächtnismaterial verwendet, wie insbesondere Nitinol oder beispielsweise auch ein Kunststoff, wie Polycarbonat. Nach dem Glühschritt erfolgt das Umformen der Grundwickelform in die gewünschte Sekundärform, wie dies in Figur 17c angedeutet ist. Diese weist beispielsweise eine der in den vorstehenden Figuren dargestellten Ausführungsformen auf oder eine beliebige andere, die für den jeweiligen Anwendungsfall der implantierbaren Einrichtung gewünscht wird. Als weiterer Fertigungsschritt ist das Glühen der Sekundärform im Ofen 110, wie es in Figur 17d angedeutet ist, vorgesehen. Hierbei wird dem Material die Sekundärform dauerhaft so eingeprägt, dass nach einem Langstrecken der Tragstruktur bzw. der implantierbaren Einrichtung zum Einbringen dieser durch einen Katheter in den Körper eines Patienten und beim späteren Herausschieben dieser aus dem Katheter diese automatisch ihre Sekundärform annimmt. Nach dem Glühen und Abkühlen wird beispielsweise die in Figur 17e dargestellte Form der implantierbaren Einrichtung erhalten. Diese kann dann beispielsweise im Bereich von VSD und ASD angewendet werden.

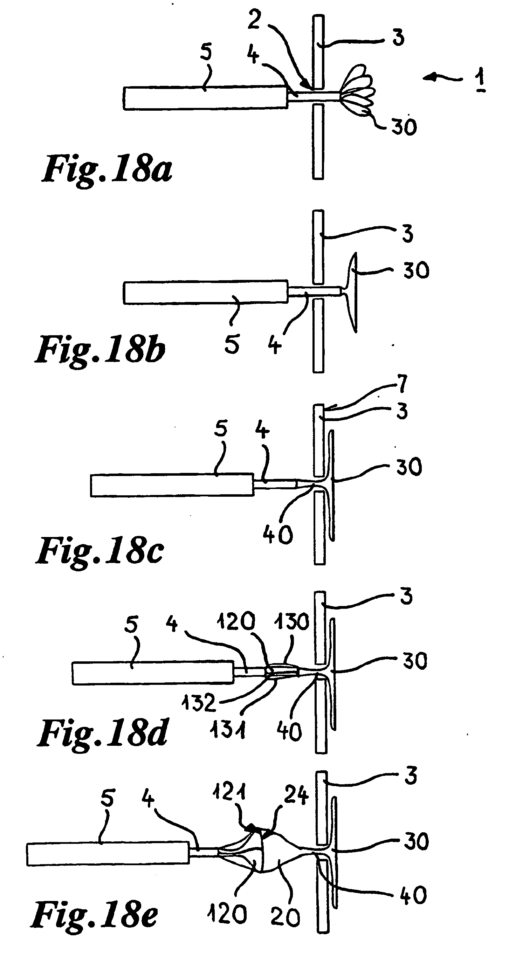

In den Figuren 18a bis 18j ist eine alternative Ausführungsform eines Abwurfs der implantierbaren Einrichtung 1 dargestellt. Hierbei wird, wie zu Figur 13a bis 13g beschrieben, die implantierbare Einrichtung in der Defektöffnung 2 in der Wandung 3 angebracht, wobei der Katheter 5 mit dem Vorschubröhrchen 4 durch die Defektöffnung 2 geschoben und die implantierbare Einrichtung 1 aus dem Vorschubröhrchen 4 herausgeschoben wird. Der distale Abschnitt 30 entfaltet sich. Das Vorschubröhrchen 4 wird aus der Öffnung 2 zurückgezogen und der Zwischenabschnitt 40 entfaltet sich. Dabei lagert sich der distale Abschnitt 30 auf der distalen Außenseite 7 der Wandung 3 an. In dem nächsten Aussetzschritt, der in Figur 18c gezeigt ist, ist die implantierbare Einrichtung vollständig aus dem Vorschubröhrchen herausgeschoben und dieses bereits aus der Öffnung 2 zurückgezogen.

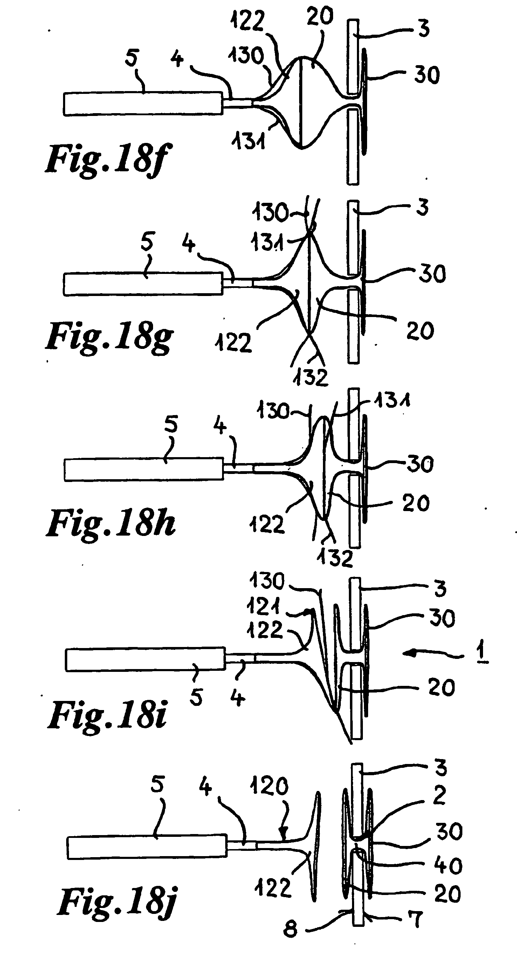

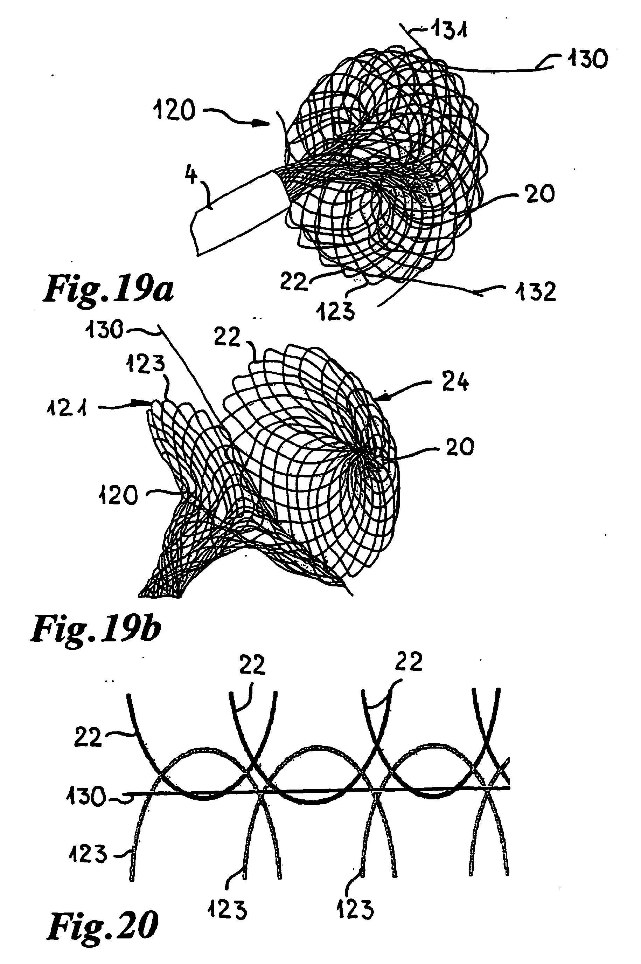

Proximalseitig bezüglich der Wandung 3 wird die implantierbare Einrichtung noch weiter aus dem Vorschubröhrchen herausgeschoben, zusammen mit einer Hilfsstruktur 120 und in dieser Ausführungsform drei Haltedrähten 130, 131, 132. Die Haltedrähte verbinden das proximale Ende 24 des proximalen Abschnitts 20 und das distale Ende 121 des distalen Abschnitts 122 der Hilfsstruktur 120 miteinander (besser in Figur 19a und 19b zu sehen). Anstelle von drei Haltedrähten können auch z.B. lediglich einer oder zwei vorgesehen werden oder auch mehr als drei. Die Verbindung kann beispielsweise wie in Figur 20 gezeigt, erfolgten. Dort werden die endseitigen Schlaufen 22, 123 oder Schlingen der beiden Tragstruktur der implantierbaren Einrichtung und Hilfsstruktur gegenüberliegend einander teilweise überdeckend aufeinander gelegt und der Haltedraht 130 wechselweise über und unter den Fäden bzw. Drähten der Schlaufen hindurchgefädelt. Die Haltedrähte sind entlang der Hilfsstruktur in dem Vorschubröhrchen geführt. Sie können auch außerhalb von diesem geführt werden. In der Ansicht nach Figur 18e sind der proximale Abschnitt 20 und die Hilfsstruktur weiter aus dem Vorschubröhrchen herausgeschoben und entfaltet. In der Ansicht nach Figur 18f ist der proximale Abschnitt 20 schon nahezu vollständig entfaltet, ebenso der distale Abschnitt 122 der Hilfsstruktur 120. Die Haltedrähte sind noch entlang der Hilfsstruktur geführt. Beim Zurückziehen der Haltedrähte und weiteren Entfalten der implantierbaren Einrichtung und Hilfsstruktur erstrecken sich die Haltedrähte in der Ansicht nach Figur 18g radial nach außen (Figur 19a). Beim weiteren Zurückziehen der Haltedrähte werden diese aus den Schlaufen 22, 123 bzw. Schlingen der Strukturen herausgezogen und stehen weiterhin von den aneinanderliegenden Rändern der Strukturen radial nach außen weg (Figur 18h). Werden die Haltedrähte weiter in das Vorschubröhrchen hinein zurückgezogen, trennen sich das distale Ende 121 der Hilfsstruktur und das proximale Ende 24 des proximalen Abschnitts der Tragstruktur der implantierbaren Einrichtung voneinander, wie in Figur 18i und 19b angedeutet. Nach dem weiteren Zurückziehen der Haltedrähte und Trennen der Strukturen voneinander ist die Tragstruktur der implantierbaren Einrichtung vollständig in der Öffnung 2 abgesetzt und entfaltet. Der proximale Abschnitt 20 lagert sich an der proximalen Außenseite 8 der Wandung 3 an (Figur 18j). Der distale Abschnitt 122 der Hilfsstruktur 120 ist ebenfalls vollständig entfaltet. Die Hilfsstruktur kann nachfolgend wieder in das Vorschubröhrchen zurückgezogen und dadurch von dem Implantationsort entfernt werden. Mit diesem Verfahren zum Absetzen einer implantierbaren Einrichtung kann vorteilhaft auch ein ggf. schwer zu entfaltender proximaler Abschnitt vollständig entfaltet werden. Es ist dabei noch nicht einmal erforderlich, dass die Enddurchmesser des entfalteten proximalen Endes der Tragstruktur der implantierbaren Einrichtung und des distalen Endes 121 der Hilfsstruktur übereinstimmen. Der Durchmesser des distalen Endes 121 kann sogar größer sein, sofern dennoch ein problemloses Entfalten des proximalen Abschnitts 20 der implantierbaren Einrichtung möglich ist.

Neben den im Vorstehenden beschriebenen und in den Figuren dargestellten Ausführungsformen, können noch weitere erdacht werden, bei denen jeweils nur ein drahtartiges Element verwendet wird, um die Tragstruktur zu bilden. Hiermit ist es jeweils möglich, insbesondere tellerartig auskragende proximale und/oder distale Abschnitte zu bilden, wobei auch zahlreiche andere Formen möglich sind.

### Bezugszeichenliste

- 1: implantierbare Einrichtung
- 2: Defektöffnung
- 3: Wandung
- 4: Vorschubröhrchen
- 5: Katheter
- 6: Führungsdraht
- 7: distale Außenseite
- 8: proximale Außenseite
- 9: Führungsdraht
- 10: drahtartiges Element
- 11: Ende
- 12: Ende
- 13: Öse
- 20: proximaler Abschnitt
- 21: Randbereich
- 22: Schlaufe
- 23: Randbereich
- 24: proximales Ende
- 25: Fläche
- 26: e-förmige Schlinge
- 27: Innenraum
- 28: Randbereich
- 29: Teilbereich
- 30: distaler Abschnitt
- 31: Randbereich
- 32: Schlaufe
- 33: Randbereich
- 34: distales Ende
- 35: Fläche
- 36: Randbereich
- 37: Öffnung
- 38: Randbereich
- 40: Zwischenabschnitt
- 41: Überdeckungsbereich
- 42: Anschlussbereich
- 50: Durchgangsöffnung
- 51: Durchgangsöffnung
- 52: Öffnung
- 60: Pfeil
- 61: Pfeil
- 62: Bereich
- 63: Längsachse
- 70: Faden
- 71: Bereich mit Faden
- 72: Membran
- 73: Membran
- 74: Arme
- 75: innere Membranfläche
- 80: erster Haltedraht
- 81: zweiter Haltedraht
- 82: Schlaufenöffnung
- 83: Schlaufe
- 90: Extraktionsdraht
- 91: Schlaufe
- 100: Hülse
- 101: Pfeile/Quetschstelle
- 102: Verbindungspunkte
- 103: Spirale
- 110: Ofen
- 120: Hilfsstruktur
- 121: distales Ende
- 122: distaler Abschnitt
- 123: Schlaufe
- 130: Haltedraht
- 131: Haltedraht
- 132: Haltedraht

## Patentansprüche

1. Implantierbare Verschlusseinrichtung (1) zur Verwendung im menschlichen und/oder tierischen Körper zum Verschluss oder Teilverschluss von Defektöffnungen, Hohlräumen, Organwegen etc., mit einer Tragstruktur, die in einem ersten Betriebszustand (Primärform) ein großes Verhältnis von Länge zu Querausdehnung entlang einer Achse (63) und in einem zweiten Betriebszustand (Sekundärform) ein kleineres Verhältnis von Länge zu Querausdehnung entlang der Achse (63) aufweist wobei die Tragstruktur einen proximalen (20) und einen distalen Absschnitt (30) aufweist, **dadurch** gekennzeichtet, dass die Tragstruktur is der Primärform aus einem einzigen drahtartigen Element (10) durch verschränkendes Wickeln und/oder Verwinden und/oder Verflechten nach Art eines Gewebes und/oder Geleges und/oder Netzers, als schlauchförmiges Element mit zwei offenen Enden geformt ist, wobei
das proximale Ende des proximalen Abschnitts (20) oder das distale Ende des distalen Abschnitts (30) der schlauchförmigen Tragstruktur der Primärform nach außen herausgezogen oden -gebogen und im äußeren Randbereich (21,31) Schlaufen oder Schlingen (22,32) von diesem gebildet sind und dieser proximale und/oder distale Abschnitt (20,30) in der Sekundärform im Wesentlichen flach scheibenförmig ist und der andere Abschnitt zunächst schreibenförmig nach aussen gebogen und nachfolgend wieder nach innen umgebogen ist und im Berreich der durchgangsöffnung, und einen Innenraum (27) umgrenzena ausgebildet ist.

2. Implantierbare Verschlusseinrichtung (1) nach Anspruch 1,
**dadurch gekennzeichnet, dass**
der proximale und der distale Abschnitt (20,30) der Tragstruktur in der Sekundärform flach und partiell so aufeinander gelegt sind, dass ein Verschluss oder Teilverschluss von seitlich durch Wandungen begrenzten Öffnungen, insbesondere im Bereich von Klappen, im menschlichen oder tierischen Körper ermöglicht ist.

3. Implantierbare Verschlusseinrichtung (1) nach einem der vorstehenden Ansprüche,
**dadurch gekennzeichnet, dass**
zumindest ein Teilbereich der implantierbaren Einrichtung (1) einfaltbar oder eingefaltet ausgeblidet ist.

4. Implantierbare Verschlusseinrichtung (1) nach einem der vorstehenden Ansprüche,
**dadurch gekennzeichnet, dass**
In der Sekundärform der Tragstruktur eine mittlere Durchgangöffnung (50) in der implantierbaren Einrichtung zum Teilverschluss einer Öffnung (2) verbleibt.

5. Implantierbare Verschlusseinrichtung (1) nach einem der vorstehenden Ansprüche,
**dadurch gekennzeichnet, dass**
eine oder mehrere Membranen (72,73) oder membranartige oder membranbildende Strukturen in die Tragstruktur eingebracht oder auf diese aufgebracht sind.

6. Verfahren zum Herstellen einer implantierbaren Verschlusseinrichtung (1) nach einem der Ansprüche 1 bis 5, **gekennzeichnet durch** die folgenden Schritte:
- Wickeln einer Tragstruktur-Grundwickelform aus einem drahtartigen Element (10) **durch** verschränkendes Wickeln und/oder Verwinden und/oder Verflechten nach Art eines Gewebes und/oder Geleges und/oder Netzes,
- Glühen der Tragstruktur-Grundwickelform zum Stabilisieren der Form,
- Umformen der Tragstruktur aus der Grundwickelform in eine gewünschte Sekundärform, bei der das proximale Ende des proximalen Abschnitts (20) und das distale Ende des distalen Abschnitts (30) nach außen herausgezogen oder -gebogen wird und im äußeren Randbereich (21,31) Schlaufen oder Schlingen (22,32) von diesem gebildet werden, und dieser proximale und/oder distale Abschnitt (20,30) in der Sekundärform im Wesentlichen flach scheibenförmig ist und der andere Abschnitt nach innen umgebogen ist und im Bereich der Durchgangöffnung, und einen Innenraum (27) umgrenzend ausgebildet ist, und
- Glühen der Tragstruktur-Sekundärform zum Stabilisieren und Einprägen der Form.

7. Verfahren nach Anspruch 6,
**dadurch gekennzeichnet, dass**
der erste Wickelschritt von Hand ausgeführt wird.

## Claims

1. An implantable occlusion means (1) for use in the body of humans and/or animals for occluding or partially occluding defective openings, cavities, organ tracts etc., with a supporting structure which in a first operating state (primary form) has a large ratio of length to transverse extent along an axis (63) and in a second operating state (secondary form) has a smaller ratio of length to transverse extent along the axis (63), the supporting structure having a proximal (20) and a distal portion (30), **characterised in that** the supporting structure in the primary form is formed as a tubular element with two open ends from a single wire-like element (10) by interwinding and/or intertwining and/or interlacing in the manner of a woven fabric and/or a laid fabric and/or a net,
the proximal end of the proximal portion (20) or the distal end of the distal portion (30) of the tubular supporting structure of the primary form being pulled or bent out outwards and in the outer edge region (21, 31) loops or hoops (22, 32) being formed thereby, and this proximal and/or distal portion (20, 30) in the secondary form being substantially flat and disc-shaped and the other portion being firstly pulled outwards in a disc shape and subsequently being bent back over inwards again and in the region of the through opening having ends of loops and hoops which are intertwined with each other, and being formed to define an inner space (27).

2. An implantable occlusion means (1) according to Claim 1,
**characterised in that**
the proximal and the distal portion (20, 30) of the supporting structure in the secondary form are placed flat and partially laid on one another such that an occlusion or partial occlusion of openings delimited laterally by walls, in particular in the region of valves, in the body of humans or animals is permitted.

3. An implantable occlusion means (1) according to one of the preceding claims,
**characterised in that**
at least a partial region of the implantable means (1) is formed to be able to be folded in, or is formed folded in.

4. An implantable occlusion means (1) according to one of the preceding claims,
**characterised in that**
in the secondary form of the supporting structure a central through opening (50) remains in the implantable means for partial occlusion of an opening (2).

5. An implantable occlusion means (1) according to one of the preceding claims,
**characterised in that**
one or more membranes (72, 73) or membrane-like or membrane-forming structures are introduced into the supporting structure or are applied thereto.

6. A method for producing an implantable occlusion means (1) according to one of Claims 1 to 5, **characterised by** the following steps:
- winding of a supporting-structure basic coil form from a wire-like element (10) by interwinding and/or intertwining and/or interlacing in the manner of a woven fabric and/or a laid fabric and/or a net,
- annealing of the supporting-structure basic coil form in order to stabilise the form,
- conversion of the supporting structure from the basic coil form into a desired secondary form, in which the proximal end of the proximal portion (20) and the distal end of the distal portion (30) are pulled or bent out outwards and in the outer edge region (21, 31) loops or hoops (22, 32) are formed thereby, and this proximal and/or distal portion (20, 30) in the secondary form is substantially flat and disc-shaped and the other portion is bent over away inwards and in the region of the through opening has ends of loops and hoops, and is formed to define an inner space (27), and
- annealing of the supporting-structure secondary form in order to stabilise and imprint the form.

7. A method according to Claim 5,
**characterised in that**
the first winding step is carried out by hand.

## Revendications

1. Dispositif d'obturation (1) implantable destiné à être utilisé dans le corps humain et/ou animal pour l'obturation ou l'obturation partielle d'ouvertures défectueuses, de cavités, de voies organiques, etc., comportant une structure de support qui, dans une première position de service (forme primaire), possède un grand rapport entre la longueur et la dimension transversale le long d'un axe (63) et, dans une deuxième position de service (forme secondaire), présente un plus petit rapport entre la longueur et la dimension transversale le long d'un axe (63), la structure de support comportant une partie proximale (20) et une partie distale (30), **caractérisé en ce que** la structure de support dans la forme primaire est réalisée avec un seul élément (10) en forme de fil pour obtenir la forme d'un élément tubulaire avec deux extrémités ouvertes au moyen d'un enroulement croisé et/ou d'une torsade et/ou d'un entrelacement à la manière d'un tissu et/ou d'un mat et/ou d'un filet, sachant que l'extrémité proximale de la partie proximale (20) ou l'extrémité distale de la partie distale (30) de la structure de support tubulaire dans la forme primaire est tirée ou courbée vers l'extérieur et, dans la zone de bordure (21, 31) extérieure, des coulisses ou boucles (22, 32) sont formées par ladite partie, et cette partie proximale et/ou distale (20, 30) dans la forme secondaire est sensiblement en forme de disque plat, et l'autre partie est d'abord tirée vers l'extérieur en forme de disque et, ensuite, est à nouveau courbée vers l'intérieur et comporte, dans la zone de l'ouverture de passage, des extrémités de coulisses et boucles entremêlées entre elles et délimitant un espace intérieur (27).

2. Dispositif d'obturation (1) implantable selon la revendication 1, **caractérisé en ce que** la partie proximale (20) et la partie distale (30) de la structure de support dans la forme secondaire sont posées l'une sur l'autre à plat et partiellement, de telle sorte qu'il est possible d'obturer ou d'obturer partiellement des ouvertures délimitées latéralement par des parois, en particulier dans la zone de valvules, dans le corps humain ou animal.

3. Dispositif d'obturation (1) implantable selon l'une quelconque des revendications précédentes, **caractérisé en ce qu'**au moins une zone partielle du dispositif (1) implantable est réalisée de manière propre à être pliée ou sous forme pliée.

4. Dispositif d'obturation (1) implantable selon l'une quelconque des revendications précédentes, **caractérisé en ce que** dans la forme secondaire de la structure de support, une ouverture de passage (50) centrale subsiste dans le dispositif implantable pour l'obturation partielle d'une ouverture (2).

5. Dispositif d'obturation (1) implantable selon l'une quelconque des revendications précédentes, **caractérisé en ce qu'**une ou plusieurs membranes (72, 73) ou des structures en forme de membranes ou formant des membranes sont insérées dans la structure de support ou posées sur celle-ci.

6. Procédé pour la réalisation d'un dispositif d'obturation (1) implantable selon l'une quelconque des revendications 1 à 5, **caractérisé par** les étapes suivantes :
- enroulement d'une forme de base pour la structure de support, réalisée dans un élément (10) en forme de fil au moyen d'un enroulement croisé et/ou d'une torsade et/ou d'un entrelacement à la manière d'un tissu et/ou d'un mat et/ou d'un filet,
- recuit de la forme de base pour la structure de support en vue de la stabilisation de la forme,
- formage de la structure de support à partir de la forme de base dans une forme secondaire souhaitée, dans laquelle l'extrémité proximale de la partie proximale (20) et l'extrémité distale de la partie distale (30) est tirée ou courbée vers l'extérieur et, dans la zone de bordure (21, 31) extérieure, des coulisses ou boucles (22, 32) sont formées par ladite partie, et cette partie proximale et/ou distale (20, 30) dans la forme secondaire est sensiblement en forme de disque plat, et l'autre partie est courbée vers l'intérieur et comporte, dans la zone de l'ouverture de passage, des extrémités de coulisses et boucles délimitant un espace intérieur (27), et
- recuit de la forme secondaire de la structure de support pour la stabilisation et l'application de la forme.

7. Procédé selon la revendication 6, **caractérisé en ce que** la première étape d'enroulement est effectuée manuellement.
